(19) Europäisches Patentamt
European Patent Office
Office européen des brevets

(11) Publication number: **0 432 442 A1**

(12) **EUROPEAN PATENT APPLICATION**

(21) Application number: **90121140.9**

(22) Date of filing: **05.11.90**

(51) Int. Cl.⁵: **C07C 275/28**, C07C 309/51, C07D 213/56, C07D 307/68, C07D 239/26, A61K 31/17

(30) Priority: **06.11.89 US 433079**
**18.09.90 US 584565**

(43) Date of publication of application:
**19.06.91 Bulletin 91/25**

(84) Designated Contracting States:
**AT BE CH DE DK ES FR GB GR IT LI LU NL SE**

(71) Applicant: **WARNER-LAMBERT COMPANY**
**201 Tabor Road**
**Morris Plains New Jersey 07950(US)**

(72) Inventor: **O'Brien, Patrick Michael**
**23 Arbor Way**
**Northville, Michigan 48167(US)**
Inventor: **Sliskovic, Drago Robert**
**4860 Cole Blvd.**
**Ypsilanti, Michigan 48197(US)**

(74) Representative: **Mansmann, Ivo**
**c/o Gödecke AG - Patentabteilung Postfach**
**569 Mooswaldallee 1-9**
**W-7800 Freiburg(DE)**

(54) **ACAT inhibitors.**

(57) This invention relates to novel compounds which are ACAT inhibitors rendering them useful in lowering blood cholesterol levels. The compounds contain two urea or thiourea, amide, or amine moieties or combinations of said moieties and have the following general formula:

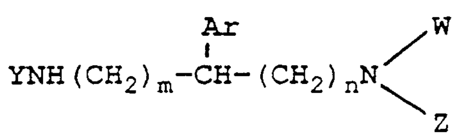

wherein Ar is an aryl group, m and n are zero or one,
W and YNH and

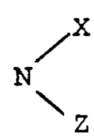

form the urea, thiourea, amide or amine moieties.

EP 0 432 442 A1

# ACAT INHIBITORS

## BACKGROUND OF THE INVENTION

This invention relates to chemical compounds having pharmacological activity, to pharmaceutical compositions which include these compounds, and to the use of such compounds for the preparation of pharmaceutical compositions. More particularly, this invention concerns certain novel compounds which inhibit the enzyme acylcoenzyme A: cholesterol acyltransferase (ACAT), pharmaceutical compositions containing these compounds useful for treating hypercholesterolemia and atherosclerosis.

In recent years the role which elevated blood plasma levels of cholesterol play in pathological conditions in man has received much attention. Deposits of cholesterol in the vascular system have been indicated as causative of a variety of pathological conditions including coronary heart disease.

Initially, studies of this problem were directed toward finding therapeutic agents which could be effective in lowering total serum cholesterol levels. It is now known that cholesterol is transported in the blood in the form of complex particles consisting of a core of cholesteryl esters plus triglycerides and an exterior consisting primarily of phospholipids and a variety of types of protein which are recognized by specific receptors. For example, cholesterol is carried to the sites of deposit in blood vessels in the form of low density lipoprotein cholesterol (LDL cholesterol) and away from such sites of deposit by high density lipoprotein cholesterol (HDL cholesterol).

Following these discoveries, the search for therapeutic agents which control serum cholesterol turned to finding compounds which are more selective in their action; that is, agents which are effective in elevating the blood serum levels of HDL cholesterol and/or lowering the levels of LDL cholesterol. While such agents are effective in moderating the levels of serum cholesterol, they have little or no effect on controlling the initial absorption of dietary cholesterol in the body through the intestinal wall.

In intestinal mucosal cells, dietary cholesterol is absorbed as free cholesterol which must be esterified by the action of enzyme acyl-CoA: cholesterol acyltransferase (ACAT) before can be packaged into the chylomicrons which are then released into the blood stream. Thus, therapeutic agents which effectively inhibit the action of ACAT prevent the intestinal absorption of dietary cholesterol into the blood stream or the reabsorption of cholesterol which has been previously released into the intestine through the body's own regulatory action.

## SUMMARY OF THE INVENTION

The present invention provides a class of compounds which have acyl-CoA: cholesterol acyltransferase (ACAT) inhibitory activity and intermediates useful in preparing said compounds having the following structure:

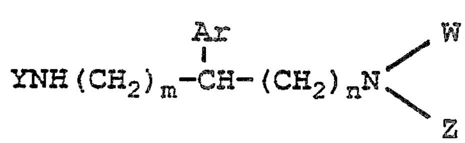

wherein each of m and n is zero or one;
wherein Ar is
(a) phenyl which is unsubstituted or is substituted with from 1 to 3 substituents selected from
alkyl having from 1 to 6 carbon atoms and which is straight or branched,
alkoxy having from 1 to 6 carbon atoms and which is straight or branched;
phenoxy,
hydroxy,
fluorine,
chlorine,
bromine,
nitro,
trifluoromethyl,

-COOH,
-COOalkyl wherein alkyl has from 1 to 4 carbon atoms,
-$NR_1R_2$ wherein
$R_1$ and $R_2$ are independently hydrogen or alkyl of from 1 to 4 carbon atoms;
(b) 1- or 2-naphthyl which is unsubstituted or substituted with
alkyl having from 1 to 6 carbon atoms and which is straight or branched;
alkoxy having from 1 to 6 carbon atoms and which is straight or branched,
hydroxy,
fluorine,
chlorine,
bromine,
nitro,
trifluoromethyl,
-COOH,
-COOalkyl wherein alkyl has from 1 to 4 carbon atoms,
-$NR_1R_2$ wherein $R_1$ and $R_2$ are as defined above; or
(c) a 5- or 6-membered monocyclic or fused bicyclic heterocycle containing at least 1 to 4 nitrogen, oxygen or sulfur atoms in at least one ring member;
wherein Y and z are independently selected from:
(a) hydrogen;

(b) 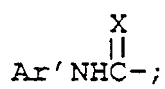

(c) 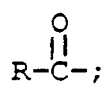

(d) R-$CH_2$; and

(e) 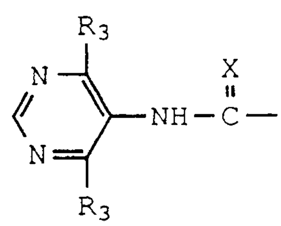

wherein $R_3$ is straight or branohed alkyl having from 1 to 4 carbon atoms or straight branched alkoxy having from 1 to 4 carbon atoms;
wherein X is oxygen or sulfur;
wherein Ar′ is selected from:
(a) phenyl which is unsubstituted or is substituted with from 1 to 3 substituents selected from
alkyl having from 1 to 6 carbon atoms and which is straight or branched,
alkoxy having from 1 to 6 carbon atoms and which is straight or branched,
phenoxy,
hydroxy,
fluorine,
chlorine,
bromine,
nitro,
trifluoromethyl,
-COOH,
-COOalkyl wherein alkyl has from 1 to 4 carbon atoms,
-$NR_1R_2$ wherein

$R_1$ and $R_2$ are independently hydrogen or alkyl of from 1 to 4 carbon atoms; and
(b) 1- or 2-naphthyl which is unsubstituted or substituted with
alkyl having from 1 to 6 carbon atoms and which is straight or branched,
alkoxy having from 1 to 6 carbon atoms and which is straight or branched,
hydroxy,
fluorine,
chlorine,
bromine,
nitro,
trifluoromethyl,
-COOH,
-COOalkyl wherein alkyl has from 1 to 4 carbon atoms,
$-NR_1R_2$ wherein $R_1$ and $R_2$ are as defined above;
wherein R is selected from:
(a) a straight or branched hydrocarbon chain having from 1 to 20 carbon atoms and which is saturated or contains from 1 to 3 double bonds;
(b) a straight or branched hydrocarbon chain having from 1 to 20 carbon atoms wherein the terminal carbon atom is substituted with chlorine; fluorine; bromine; straight or branched lower alkoxy having from 1 to 4 carbon atoms; straight or branched thioalkoxy having from 1 to 4 carbon atoms; a $-COOR_4$ group wherein $R_4$ is hydrogen or a straight or branched alkyl having from 1 to 4 carbon atoms; an $-NR_5R_6$ group wherein $R_5$ and $R_6$ are independently hydrogen or lower alkyl having from 1 to 4 carbon atoms wherein said alkyl is unsubstituted or is substituted with hydroxy, or wherein $-NR_5R_6$ taken together form a monocyclic heterocyclic group selected from pyrrolidino, piperidino, piperazino, or piperazino substituted in the 4-position with a lower alkyl having from 1 to 4 carbon atoms or $-COOR_4$ wherein $R_4$ has the meaning defined above; and
(c) a 5- or 6-membered monocyclic or fused bicyclic heterocycle containing at least 1 to 4 nitrogen, oxygen or sulfur atoms in at least one ring member;
(d) the group

$$-(CH_2)_t-\underset{R_8}{\overset{R_7}{\underset{|}{\overset{|}{C}}}}-(CH_2)_p-R_9$$

wherein t is zero to 4; p is zero to 4 with the proviso that the sum of t and p is not greater than 5; $R_7$ and $R_8$ are independently selected from hydrogen or straight or branched alkyl having from 1 to 6 carbon atoms, or when $R_7$ is hydrogen, $R_8$ can be selected from the groups defined for $R_9$; and $R_9$ is phenyl or phenyl substituted with from 1 to 3 substituents selected from straight or branched alkyl having from 1 to 6 carbon atoms, straight or branched alkoxy having from 1 to 6 carbon atoms, straight or branched thioalkoxy having from 1 to 6 carbon atoms, phenoxy, hydroxy, fluorine, chlorine, bromine, nitro, trifluoromethyl, -COOH, COOalkyl wherein alkyl has from 1 to 4 carbon atoms, or $NR_5R_6$ wherein $R_5$ and $R_6$ having the meanings defined above;
(e) phenyl or phenyl substituted with from 1 to 3 substituents selected from straight or branched alkyl having from 1 to 6 carbon atoms, straight or branched alkoxy having from 1 to 6 carbon atoms, straight or branched thioalkoxy having from 1 to 6 carbon atoms, phenoxy, hydroxy, fluorine, chlorine, bromine, nitro, trifluoromethyl, -COOH, COOalkyl wherein alkyl has from 1 to 4 carbon atoms, or $-NR_5R_6$ wherein $R_5$ and $R_6$ have the meanings defined above; and
(f) 1- or 2-naphthyl or 1- or 2-naphthyl substituted with from 1 to 3 substituents selected from straight or branched alkyl having from 1 to 6 carbon atoms, straight or branched alkoxy having from 1 to 6 carbon atoms, hydroxy, chlorine, fluorine, bromine, nitro, trifluoromethyl, -COOH, COOalkyl wherein alkyl has from 1 to 4 carbon atoms, or $-NR_5R_6$ wherein $R_5$ and $R_6$ have the meanings defined above;
wherein W is selected from:
(a) hydrogen;
(b) a straight or branched hydrocarbon chain having from 1 to 20 carbon atoms and which is saturated or contains from 1 to 3 double bonds;
(c) a straight or branched hydrocarbon chain having from 1 to 20 carbon atoms wherein the terminal carbon atom is substituted with chlorine; fluorine; bromine; straight or branched lower alkoxy having from 1 to 4

carbon atoms; straight or branched thioalkoxy having from 1 to 4 carbon atoms; a $-COOR_4$ group wherein $R_4$ has the meaning defined above; an $-NR_5R_6$ group wherein $R_5$ and $R_6$ have the meaning defined above;
(d) the group

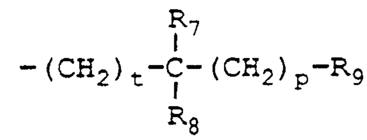

wherein t, p, $R_7$, $R_8$, and $R_9$ have the meanings defined above;
(e) phenyl or phenyl substituted with from 1 to 3 substituents selected from straight or branched alkyl having from 1 to 6 carbon atoms, straight or branched alkoxy having from 1 to 4 carbon atoms, straight or branched thioalkoxy having from 1 to 4 carbon atoms, phenoxy, hydroxy, $SO_3H$, fluorine, chlorine, bromine, nitro, trifluoromethyl, -COOH, COOalkyl wherein alkyl has from 1 to 4 carbon atoms, or $-(CH_2)_s-NR_5R_6$ wherein s is zero to 2, and $R_5$ and $R_6$ have the meanings defined above; and
(f) the group

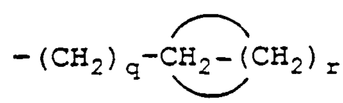

wherein q is zero to 2 and r is 2 to 6; or a pharmaceutically acceptable salt and N-oxides thereof; with the provisos:
(a) m and n are not zero at the same time;
(b) where both Z and W are the group

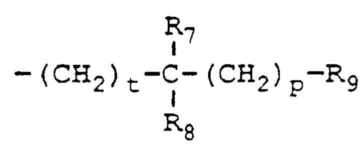

$R_8$ and $R_9$ are not the same;
(c) each of Y, Z, and W are not hydrogen at the same time; and
(d) when Ar represents a 5- or 6-membered monocyclic or fused bicyclic heterocycle, one of m or n is zero.

In addition to being pharmaceutically useful compounds, the compounds of Formula I wherein Y, Z or W is hydrogen also can be intermediates to prepare other compounds of Formula I which will be apparent from the general description of the preparation of the compounds and the specific examples.

## DETAILED DESCRIPTION OF THE INVENTION

The present invention provides a novel class of compounds which contain one or two moieties selected from amine, amide, urea, and thiourea moieties or combinations thereof which are ACAT inhibitors rendering them useful in treating hypercholesterolemia and atherosclerosis.

Illustrative examples of straight or branched saturated hydrocarbon chains having from 1 to 20 carbon atoms include methyl, ethyl, n-propyl, isopropyl, n-butyl, iso-butyl, tert-butyl, n-pentyl, isopentyl, n-hexyl, n-heptyl, n-octyl, n-undecyl, n-dodecyl, n-hexadecyl, 2,2-dimethyldodecyl, 2-ethyltetradecyl, and n-octadecyl groups.

Illustrative examples of straight or branched hydrocarbon chains having from 1 to 20 carbon atoms and having from 1 to 3 double bonds include ethenyl, 2-propenyl, 2-butenyl, 3-pentenyl, 2-octenyl, 5-nonenyl, 4-undecenyl, 5-heptadecenyl, 3-octadecenyl, 9-octadecenyl, 2,2-dimethyl-11-eicosenyl, 9,12-octadecadienyl, and hexadecenyl.

Straight or branched alkoxy groups having from 1 to 6 carbon atoms include, for example, methoxy, ethoxy, n-propoxy, t-butoxy, and pentyloxy.

Illustrative of straight or branched thioalkoxy groups having from 1 to 6 carbon atoms are methylthio,

ethylthio, *n*-propylthio, isopropylthio, and butylthio. The thioalkoxy group may also be referred to as alkylthio.

A 5- or 6-membered monocyclic or fused bicyclic heterocycle is a monocyclic or fused bicyclic aromatic ring containing at least 1 to 4 heteroatoms in at least one ring, such as nitrogen, oxygen, or sulfur or a combination thereof. Such a heterocyclic group includes, for example, thienyl, benzothienyl, furanyl, benzofuranyl, pyridyl, pyrimidinyl, pyridazinyl, pyrazinyl, pyrrolyl, pyrazolyl, isothiazolyl, oxazolyl, isoxazolyl, triazolyl, tetrazolyl, imidazolyl, benzothiazolyl, indolyl, quinolinyl, isoquinolinyl, or N-oxides of heterocycle containing a nitrogen atom.

More specifically, such a heterocycle may be a 2-or 3-thienyl ; 2- or 3-furanyl; 2-, or 3-, or 4-pyridyl or -pyridyl-N-oxide; 2-, 4-, or 5-pyrimidinyl; 3- or 4-pyridazinyl; 2-pyrazinyl; 2-pyrazinyl-N-oxide; 2- or 3-pyrrolyl; 3-, 4-, or 5-pyrazolyl; 3-, 4-, or 5-oxazolyl; 3-, 4-, or 5-isoxazolyl; 3-, 4-, or 5-isothiazolyl; 5-tetrazolyl; 3- or 5-(1,2,4,-)triazolyl; 4- or 5-(1,2,3-)triazolyl; 2-, 4-, or 5-imidazolyl; 2-, 3-, 4-, 5-, 6-, or 7-indolyl; 2-, 3-, 4-, 5-, 6-, 7-, or 8-quinolinyl; 1-, 3-, 4-, 5-, 6-, 7-, or 8-isoquinolinyl; 2-, 4-, 5-, 6-, or 7-benzothiazolyl; or 2-, 3-, 4-, 5-, 6-, or 7-benzothienyl.

Preferred compounds of this invention are those wherein Ar is phenyl or Ar′ is phenyl or substituted phenyl and more preferably wherein Ar′ is phenyl substituted on the 2,6-positions. Other preferred compounds of this invention are those wherein W is hydrogen and Z and Y are independently selected from

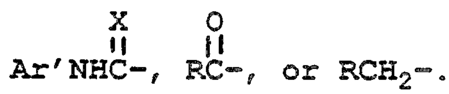

Pharmaceutically acceptable salts of the compounds of Formula I are also included as a part of the present invention.

The acid salts may be generated from the free base by reaction of the latter with one equivalent of a suitable nontoxic, pharmaceutically acceptable acid, followed by evaporation of the solvent employed for the reaction and recrystallization of the salt if required. The free base may be recovered from the acid salt by reaction of the salt with an aqueous solution of a suitable base such as sodium carbonate, sodium bicarbonate, potassium carbonate, sodium hydroxide, and the like.

Suitable acids for forming acid salts of the compounds of this invention include, but are not necessarily limited to acetic, benzoic, benzenesulfonic, tartaric, hydrobromic, hydrochloric, citric, fumaric, gluconic, glucuronic, glutamic, lactic, malic, maleic, methanesulfonic, pamoic, salicylic, stearic, succinic, sulfuric, and tartaric acids. The class of acids suitable for the formation of nontoxic, pharmaceutically acceptable salts is well known to practitioners of the pharmaceutical formulation arts (see, for example, Stephen N. Berge, et al, *J Pharm Sciences* 66:1-19 (1977)).

The compounds of the present invention may also exist in different stereoisomeric forms by virtue of the presence of asymmetric centers in the compound. The present invention contemplates all stereoisomeric forms of the compounds as well as mixtures thereof, including racemic mixtures.

Further, the compounds of this invention may exist in unsolvated as well as solvated forms with pharmaceutically acceptable solvents such as water, ethanol and the like. In general, the solvated forms are considered equivalent to the unsolvated forms for the purposes of this invention.

As shown by the data presented below in Table 1, the compounds of the present invention are potent inhibitors of the enzyme acyl-CoA: cholesterol acyltransferase (ACAT), and are thus effective in inhibiting the esterification and transport of cholesterol across the intestinal cell wall. The compounds of the present invention are thus useful in pharmaceutical formulations for the treatment of hypercholesterolemia or atherosclerosis.

The ability of representative compounds of the present invention to inhibit ACAT was measured using an in vitro test more fully described in F. J. Field and R. G. Salone, *Biochemica et Biophysica* 712:557-570 (1982). The test assesses the ability of a test compound to inhibit the acylation of cholesterol by oleic acid by measuring the amount of radiolabeled cholesterol oleate formed from radiolabeled oleic acid in a tissue preparation containing rabbit intestinal microsomes.

The data appear in Table 1 where they are expressed in $IC_{50}$ values; i.e., the concentration of test compound required to inhibit 50% expression of the enzyme.

TABLE 1

| Compound of Example | $IC_{50}$ (μM) |
|---|---|
| 4 | 0.53 |
| 5 | 0.051 |
| 7 | 0.15 |
| 8 | 0.11 |
| 12 | 0.08 |
| 9 | 0.38 |
| 10 | 0.39 |

In one in vivo screen designated APCC, male Sprague-Dawley rats (200 to 225 g) were randomly divided into treatment groups and dosed at 4 PM with either vehicle (CMC/Tween) or suspensions of compounds in vehicle. The normal chow diet was then replaced with the PCC diet with either 1% or 0.5% cholic acid, as indicated. The rats consumed this diet ad libitum during the night and were sacrificed at 8 AM to obtain blood samples for cholesterol analysis using standard procedures. Statistical differences between mean cholesterol values for the same vehicle were determined using analysis of variance followed by Fisher's least significant test. The results of this trial for representative compounds of the present invention appear in Table 2.

TABLE 2

| Compound of Example | % Change (mg/dl) |
|---|---|
| 5 | -53 |
| 8 | -25 |
| 9 | -36 |
| 11 | -54 |

In therapeutic use as agents for treating hypercholesterolemia or atherosclerosis, the compounds of Formula I or pharmaceutically acceptable salts thereof are administered to the patient at dosage levels of from 250 to 3000 mg per day. For a normal human adult of approximately 70 kg of body weight, this translates into a dosage of from 5 to 40 mg/kg of body weight per day. The specific dosages employed, however, may be varied depending upon the requirements of the patient, the severity of the condition being treated, and the activity of the compound being employed. The determination of optimum dosages for a particular situation is within the skill of the art.

For preparing the pharmaceutical compositions from the compounds of this invention, inert, pharmaceutically acceptable carriers can be either solid or liquid. Solid form preparations include powders, tablets, dispersible granules, capsules, and cachets.

A solid carrier can be one or more substances which may also act as diluents, flavoring agents, solubilizers, lubricants, suspending agents, binders, or tablet disintegrating agents; it can also be an encapsulating material.

In powders, the carrier is a finely divided solid which is in a mixture with the finely divided active component. In tablets, the active component is mixed with the carrier having the necessary binding properties in suitable proportions and compacted in the shape and size desired.

Powders and tablets preferably contain between about 5% to about 70% by weight of the active ingredient. Suitable carriers are magnesium dicarbonate, magnesium stearate, talc, lactose, sugar, pectin, dextrin, starch, tragacanth, methyl cellulose, sodium carboxymethyl cellulose, a low-melting wax, cocoa butter, and the like.

The term "preparation" is intended to include the formulation of the active compound with encapsulating material as a carrier providing a capsule in which the active component (with or without other carriers) is

surrounded by a carrier, which is thus in association with it. In a similar manner cachets are also included.

Tablets, powders, cachets, and capsules can be used as solid dosage forms suitable for oral administration.

Liquid form preparations include solutions, suspensions, or emulsions suitable for oral administration. Aqueous solutions for oral administration can be prepared by dissolving the active compound in water and adding suitable flavorants, coloring agents, stabilizers, and thickening agents as desired. Aqueous suspensions for oral use can be made by dispersing the finely divided active component in water together with a viscous material such as natural or synthetic gums, resins, methyl cellulose, sodium carboxymethylcellulose, and other suspending agents known to the pharmaceutical formulation art.

Preferably, the pharmaceutical preparation is in unit dosage form. In such form, the preparation is divided into unit doses containing appropriate quantities of the active component. The unit dosage form can be a packaged preparation containing discrete quantities of the preparation, for example, packeted tablets, capsules, and powders in vials or ampoules. The unit dosage form can also be a capsule, cachet, or tablet itself, or it can be the appropriate number of these packaged forms.

The compounds of this invention are prepared by procedures generally well known in the art and set forth in Charts I and II hereof. In each of Charts I and II the various symbols Ar, Ar′, R, W, and X have the meanings defined in Formula 1 and q is zero or one. Compounds of Formulas 4 to 7 and 10 to 15 represent compounds of the invention as defined by general Formula I. The compounds are urea (thiourea), amides (4,12); urea (thiourea), amines (5,7,13); urea (thiourea), ureas (thioureas) (6); amide, amides (10); amine, amines (11,15); amide amines (14).

To form compounds containing a urea or thiourea moiety the appropriate amine is treated with an aryl isocyanate or an aryl thioisocyanate of the formula Ar′NCX wherein Ar′ and X have the meanings defined in Formula I at room temperature in methylene chloride optionally in the presence of triethylamine. To form compounds containing an amide moiety the appropriate amine is treated with an acid anhydride of the formula $(RCO)_2O$ wherein R has the meaning defined in Formula I. Additionally, an appropriate acid, $RCO_2H$ or acid halide RCOhalo wherein halo is, e.g., chlorine, may also be used. The reaction is carried out at room temperature in THF and triethylamine. In preparing compounds wherein R is heteroaryl an appropriate heteroarylcarboxylic acid is used with a coupling agent such as carbonyldiimidazole in THF or dicyclohexylcarbodiimide in methylene chloride.

The amine-containing compounds are formed by reducing the corresponding amide via a metal (aluminum) hydride reduction at reflux in toluene or by alkylating a primary amine containing compound (3,9). This alkylation is achieved by reacting compounds (3) or (9) with an aldehyde of the formula WCHO wherein W has the meaning defined in Formula I to form an imine which is reduced to the amine (7) or (14) via a metal hydride reducing agent in situ. The reaction is carried out in a lower alcohol solvent and the reaction may require heating if hindered amines are being prepared. It is apparent that compounds (7) and (14) as well as compounds (5) and (11) could be further alkylated as described above to give compounds of Formula I wherein W and Z are both other than hydrogen.

The nitriles (2,8) are hydrogenated to the amines using Raney nickel in methanolic ammonia at room temperature under 50 p.s.i.

Additionally, it is apparent that compounds (1) can be alkylated with an aldehyde, WCHO, as described above, followed by reduction of the nitrile to the amine which can be further alkylated as described above.

Compounds of Formula I wherein R represents an alkyl group having from 1 to 6 carbon atoms wherein the terminal carbon is substituted with halogen, methoxy, or $NR_2R_3$ are prepared by acylating the appropriate amine using ω-bromoacyl chloride to afford a compound wherein R is $-(CH_2)_mBr$ wherein m is an integer of from 1 to 6. The ω-bromoalkyl containing compound can be subjected to various nucleophilic substitutions to give the corresponding compounds wherein the terminal carbon is substituted with methoxy, $NR_2R_3$ or other halogen atoms. The methoxy containing compound is obtained by treating the bromo compound with methanol and sodium hydroxide. The $NR_2R_3$ containing compounds are obtained, e.g., by treating the bromo compound with ammonia gas to give the corresponding ω-$NH_2$ compound, or with dimethylamine gas to give the ω-$N(CH_3)_2$ compound or with an excess of an appropriate amine in a lower alcohol solvent at elevated temperature, e.g., 80 to 95° C to give the corresponding ω-$NR_2R_3$ containing compounds.

Compounds of this invention wherein Y and Z represents the group

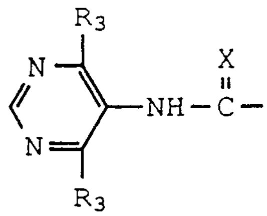

are prepared as outlined in Charts I and II only a pyrimidine isocyanate or a pyrimidine thioisocyanate of Formula II or Formula III (see Chart III) is substituted for Ar′NCX. The reactants of Formula II and III are prepared or set forth in Chart III. The dichloro pyrimidine compound (I) is commercially available and is treated with two equivalents of an appropriate Grignard reagent R¹¹MgX wherein X is chloro or bromo and R¹¹ is a straight or branched lower alkyl group having 1 to 4 carbon atoms to give the dialkyl substituted pyrimidine (2) which is reduced to the amine (3) by catalytic hydrogenation. Also, the dichloropyrimidine compound may be treated with a sodium alkoxide in methanol to give the dialkoxy pyrimidine compounds (4) which are reduced to amines (5) by catalytic hydrogenation. The amines (3) and (5) are then treated with phosgene or thiophosgene to give the reactants designated Formulas II and III.

In Charts I and II the compounds of formula (1) wherein Ar is a 5- or 6-membered monocyclic or fused bicyclic heterocycle containing at least 1 to 4 nitrogen, oxygen or sulfur in at least one ring member are prepared via a Strecker reaction whereby an appropriate heteroaldehyde is treated with potassium cyanide and ammonia in a solvent such as acetic acid and methanol. Similarly, compounds of Formula I wherein W is a phenyl or substituted phenyl moiety are prepared by reacting an aryl aldehyde or a heteroaryl aldehyde with potassium cyanide and aniline or substituted aniline in acetic acid and methanol to give N-phenyl-α-phenylaminoacetonitrile which can be substituted for formula (1) in Charts I and II. This procedure is exemplified in Example 19.

The procedure outlined in Charts I, II, and III give the final products of this invention as racemic mixtures. It is apparent that the compounds of this invention have an asymmetric carbon atom as noted by (*) in Formula I. The enantiomers of the compounds of Formula I wherein one of m or n is one and wherein Ar is other than a 5- or 6-membered monocyclic or fused heterocycle are prepared as set forth in Chart IV by procedures generally well known in the art. In Chart IV the various reactants have their usual chemical meanings with TEA being triethylamine, LAH being lithium aluminum hydride, THF being tetrahydrofuran, DMF being dimethylformamide. The chiral amine (16) is protected, e.g., butyloxycarbonyl anhydride after which the alcohol moiety is converted to an amine indirectly. First the alcohol is treated with methanesulphonyl chloride to give a mesylate (18) which is treated with sodium azide to give the alkyl azide (19). The azide can be reduced using metal hydride such as lithium aluminum hydride to give the amine (20). The acylation of compounds (20) is carried out as described above in Charts I and II, and in addition to the acid anhydride, an acyl halide, or the appropriate acid may be used. The treatment of compounds (20) with an arylisocyanate or aryl thioisocyanate is carried out as described in Charts I and II. The compounds of Formulas (22) and (24) can be treated in the same manner as compounds (3) in Chart I or compounds (9) in Chart II to give the corresponding amides (28) and (25), ureas or thioureas (29) and (26) or amines (30) and (27). Compounds (28), (29), (30), and (34) can be reduced to give compounds of formulas (31), (32), (33), and (34) as generally described herein above in Charts I and II. It is apparent that compounds (20) could also be alkylated after which, the butyloxy carbonyl (BOC) protecting group is removed to give a free amine which could also be alkylated as generally described above. Refer to the Formula Chart for compounds of formulas (25) to (34) wherein the various substituents R, Ar, X, Ar′, and W have the meanings defined in Formula I except that Ar is other than a 5- or 6-membered monocyclic or fused heterocycle.

## EXAMPLE 1

Preparation of N′,N″-bis[2,6-bis(1-methylethyl)phenyl]-N,N″-(2-phenyl-1,3-propanediyl) bis-urea

(a) N-[2,6-bis(1-methylethyl)phenyl]-N]-(2-cyano-2-phenylethyl)urea

To a $CH_2Cl_2$ (10 mL) suspension 0.76 g (0.0038 mole) of 2-cyano-2-phenethylamine•HCl (see U.S. 4,760,089) was added 0.55 mL (0.0038 mole) triethylamine. The solid dissolved on gentle warming, and 0.82 mL (0.0038 mole) 2,6-diisopropylphenylisocyanate was added and the reaction mixture was stirred at

room temperature for 3 hours. The mixture was partitioned between water and $CH_2Cl_2$. The organic layer was washed with water and saturated aqueous NaCl. The organic layer was dried over $MgSO_4$, filtered, and concentrated in vacuo. The white solid was triturated with ether, affording a white solid, m.p. 191-195 ° C.

(b) N-(3-amino-2-phenylpropyl)-N′-[2,6-bis(1-methylethyl)phenyl]urea

A mixture of 0.7 g (0.02 mole) of the urea from (a), 1 g Raney nickel, and 75 mL of methanolic ammonia was hydrogenated at 50 psi. This was then concentrated in vacuo to give the title compound (b) as a white green foam and was used without further purification in the next step.

(c) N′,N‴-bis[2,6-bis(1-methylethyl)phenyl]N,N″-(2-phenyl-1,3-propanediyl)bis-urea

The product from (b) (0.32 g, 0.009 mole) was dissolved in 10 mL $CH_2Cl_2$ at room temperature under $N_2$, followed by addition of 0.2 mL (0.009 mole) 2,6-diisopropylphenylisocyanate at room temperature with stirring. The solution was stirred for 24 hours, then concentrated in vacuo, and the resulting solid was recrystallized from ethyl acetate/methylene chloride, m.p. >220 ° C.
MS (EI), m/e 557.44
IR (KBr) $\nu$max, 3400, 2980, 2300, 1650, 1530, 1490, 1230 $cm^{-1}$
NMR($CDCl_3$) $\delta$ 7.25-7.0 (m, 11H), 3.4 (m, 4H), 3.1 (m, 4H), 3.0 (m, 1H), 1.1 (m, 24H) ppm.

## EXAMPLE 2

N-[3-[[[[2,6-bis(1-methylethyl)phenyl]amino]carbonyl]amino]-2-phenylpropyl] heptanamide

(a) N-(2-cyano-2-phenylethyl)heptanamide

(b) N-(3-amino-2-phenylpropyl)heptanamide

To a suspension of 0.96 g (0.0052 mole) of 2-cyano-2-phenylethylamine•HCl in 20 mL THF at room temperature under $N_2$ was added triethylamine (1.46 mL). This suspension was stirred vigorously for 1 hour, after which it was filtered and 1.38 mL (0.0052 mole) of heptanoic anhydride was added to the filtrate under $N_2$ with stirring. The mixture was stirred at room temperature for 24 hours, after which 10 mL of 1N NaOH was added with vigorous stirring for 30 minutes. The layers were separated, and the organic portion was washed extensively with water, saturated aqueous NaCl, dried over $MgSO_4$, and filtered. Concentration of the solvent in vacuo gave compound (a), 0.7 g (0.0027 mole) of which was hydrogenated with 75 mL methanolic ammonia and 0.5 g Raney nickel at 50 psi to give 0.71 g of compound (b).

The amino amide (b) was dissolved in $CH_2Cl_2$ at room temperature under $N_2$, followed by the addition of 2,6-diisopropylphenylisocyanate (0.58 mL, 0.0027 mole) in one portion. The solution was stirred at room temperature for 4 hours, affording a solid precipitate that was collected by filtration and washed with cold $CH_2Cl_2$ and acetone to give the title compound, m.p. 195-197 ° C.
MS (EI), m/e 465.4
IR (KBr), $\nu$max, 3200, 3000, 1630, 1550, 1490, 1250 $cm^{-1}$
NMR ($CDCl_3$) $\delta$ 7.1-7.3 (m, 8H), 7.0 (m, 2H), 6.5 (br.s, 1H), 5.8 (br.s, 1H), 3.6 (m, 2H), 3.4 (m, 2H), 3.1 (m, 2H), 2.9 (m, 1H), 2.1 (tr, 2H), 1.0-1.6 (m, 20H), 0.85 (tr, 3H) ppm.

## EXAMPLE 3

N-[3-[[[[2,6-bis(1-methylethyl)phenyl]amino]carbonyl]amino]-2-phenylpropyl]dodecanamide

(a) N-(2-cyano-2-phenylethyl)dodecanamide

(b) N-(3-amino-2-phenylpropyl)dodecanamide

To a suspension of 2 g (0.011 mole) of 2-cyano-2-phenethylamine•HCl in THF at room temperature under $N_2$ was added 3.1 mL (0.022 mole) of triethylamine, and the mixture was stirred at room temperature for 1 hour, after which the suspension was filtered and 2.4 g (0.011 mole) lauroyl chloride was added via a

syringe to the filtrate. A white solid precipitated and was stirred overnight at room temperature. Water was added to the suspension and it was stirred for 15 minutes, after which ethyl acetate was added and the aqueous phase separated. The organic layer was washed with water, saturated aqueous NaCl, dried over $MgSO_4$, filtered, and concentrated in vacuo to give 3.17 g of the above compound (a). Compound (a) was hydrogenated with 100 mL methanolic ammonia and 1.5 g Raney nickel at 50 psi to give 3.1 g of compound (b).

(c) N-[3-[[[[2,6-bis(1-methylethyl)phenyl)amino)carbonyl]amino]-2-phenylpropyl]dodecanamide

The amino amide (b) above (0.0093 mole) was dissolved in $CH_2Cl_2$ at room temperature under $N_2$, followed by the addition of 2.0 mL (0.0093 mole) of 2,6-diisopropylphenylisocyanate in one portion. The solution was stirred at room temperature overnight, after which water was added and the layers separated. The organic layer was washed with water, saturated aqueous NaCl, dried over $MgSO_4$, filtered, and concentrated in vacuo. The solid was recrystallized from acetone to give the title compound.
m.p. 157-159 °C.
MS (Cl) m/e 536.31
IR (KBr) νmax 3250, 2950, 1630, 1550, 1490, 1200 $cm^{-1}$
NMR($CDCl_3$) δ 7.1-7.4 (m, 8H), 6.5 (tr, 1H), 5.9 (s, 1H), 3.6 (m, 2H), 3.4 (m, 2H), 3.2 (m, 2H), 2.9 (m, 1H, 2.1 (tr, 2H), 1.0-1.8 (m, 30H), 0.96 (m, 3H) ppm.

Following the general procedure of Example 3 above, only substituting an appropriate amount of benzoyl chloride or valeryl chloride for lauroyl chloride the following compounds are obtained respectively:
N-[3-[[[[2,6-bis(1-methylethyl)phenyl]amino]carbonyl] amino]-2-phenylpropyl]benzamide, m.p. 156-160 °C.
N-[3-[[[[2,6-bis-(1-methylethyl)phenyl]amino]carbonyl]amino]-2-phenylpropyl]pentanamide, m.p. 176-177 °C.

Following the general procedure of Example 3 above, only substituting an appropriate amount of 2-cyano-2-(m-hydroxyphenyl)ethylamine (U.S. 4,760,089) for 2-cyano-2-phenethylamine and substituting an appropriate amount of benzoyl chloride for lauroyl chloride the following compound is obtained:
N-[3-[[[[2,6-bis-(1-methylethyl)phenyl]amino]carbonyl]amino-2-(m-hydroxyphenyl)propyl]benzamide, foam.

## EXAMPLE 4

(±)-N-[2-[[[[2,6-bis(1-methylethyl)phenyl]amino]carbonyl]amino]-1-phenylethyl]benzenecarboxamide

Into 500 mL of THE was stirred 25 g (0.148 mole) of (±)-2-phenylglycinonitrile•HCl after which 24.9 g (0.296 mole) of triethylamine (TEA) was added. The mixture was stirred for 15 minutes, filtered, and the filtrate transferred to a 1-L, three-neck flask. 20.8 g (0.148 mole) of benzoyl chloride was added in a steady stream and the mixture was stirred for 1 hour at room temperature. The TEA•HCl was removed by filtration and washed with THF. The filtrate was diluted with 800 mL ethyl acetate and washed with 150 mL of HCl (1N), 150 mL NaOH (1N), and 150 mL of saturated aqueous NaCl. The solution was dried over $MgSO_4$, filtered, and concentrated to dryness to give N-(cyanophenylmethyl)benzamide. 30.2 g (0.12 mole) of the benzamide in 300 mL of methanolic ammonia was treated with 15 g Raney nickel under 50 psi. The methanol was concentrated to dryness, leaving a yellow solid which was dried overnight in vacuo at room temperature to give N-(2-amino-1-phenylethyl)benzamide.

The amine benzamide (28.7 g, 0.12 mole) was dissolved in 700 mL of THF followed by the addition of 24.3 g (0.12 mole) 2,6-diisopropylphenylisocyanate in one portion. The reaction was stirred for 4 hours. The precipitate was collected by filtration and washed with hexane. The product was dried in vacuo at 40 °C for 3 hours to give the title compound, m.p. 249-250 °C.

Following the general procedure of Example 4, only substituting the acid chloride listed below for benzoyl chloride, the respective products listed in Table 3 were obtained.

TABLE 3

| Acid Chloride | Product | m.p./° C |
|---|---|---|
| 2,6-dichlorobenzoyl chloride | (±)-N-[2-[[[[2,6-bis-(1-methylethyl)phenyl]-amino]carbonyl]amino]-1-phenylethyl]-2,6-di-chlorobenzenecarboxamide | 230-232 |
| 4-methylbenzoyl chloride | (±)-N-[2-[[[[2,6-bis-(1-methylethyl)phenyl]-amino]carbonyl]amino]-1-phenylethyl]-4-methylbenzenecarboxamide | 247-249 |
| 4-methoxybenzoyl chloride | (±)-N-[2-[[[[2,6-bis-(1-methylethyl)phenyl]-amino]carbonyl]amino]-1-phenylethyl]-4-methoxybenzenecarboxamide | 240-242 |

Also by following the general procedure of Example 4, only substituting an appropriate amount of hexanedioic acid, mono methyl ester for benzoyl chloride and using carbodiimide as a coupling agent, the following compound was obtained:

(±)-6-[2-[[[[[2,6-bis(1-methylethyl)phenyl]amino]carbonyl]amino]-1-phenylethyl]amino]carbonyl]hexanoic acid methyl ester, m.p. 176-178 °C.

EXAMPLE 5

(±)-N-(2,6-bis(1-methylethyl)phenyl]-N′-[2-phenylmethylamino)-2-phenylethyl]urea and the hydrochloride salt

To a three-neck, 2-L flask equipped with an overhead stirrer, reflux condenser and addition funnel were added 27.0 g (0.06 mole) of (±)-N-[2-[[[[2,6-bis(1)-methylethyl)phenyl]amino]carbonyl]amino]-1-phenylethyl]-benzenecarboxamide and 400 mL toluene. To the rapidly stirred slurry was added under $N_2$ sodium bis (2-methoxyethoxy) aluminum hydride (Red-Al) (3.4 M solution in toluene) in a steady stream. The solution was brought to reflux for 2 hours. The solution was cooled in an ice bath followed by the cautious addition of 250 mL NaOH (1N). The mixture was diluted with 500 mL of ethyl acetate and the layers were separated. The organic solution was dried over $MgSO_4$, filtered, and concentrated to dryness to give (±)-N-[2,6-bis(1-methylethyl)phenyl]-N′-[2-(phenylmethylamino)-2-phenylethyl]urea, m.p. 186-187 °C.

The amino urea (1.0 g, 2.3 mmol) was dissolved in 30 mL THF and a solid immediately precipitated upon addition of HCl (g). The gelatinous solid was collected by filtration, washed with THF, and dried overnight in vacuo at 40 °C to give the hydrochloride salt, m.p. 232-233 °C.

Following the general procedure of Example 5, only substituting an appropriate amount of (±)-N-[2-[[[[2,6-bis(1-methylethyl)phenyl]amino]carbonyl]amino]-2-phenylethyl]benzamide or (±)-N-[2-[[[[2,6-bis(1-methylethyl)phenyl]amino]carbonyl]amino]-2-phenylethyl]-6-methoxyhexanamide for (±)-N-[2-[[[[2,6-bis-(1-methylethyl)phenyl]amino]carbonyl]amino]-1-phenylethyl]benzenecarboxamide the following respective products were obtained:

(±)-N-[2,6-bis(1-methylethyl)phenyl]-N′-[2-(phenylmethylamino)-1-phenylethyl]urea;

(±)-N-[2,6-bis(1-methylethyl)phenyl]-N′-[2-(6-methoxyhexylamino)-1-phenylethyl]urea.

EXAMPLE 6

(±)-N-[2-[[[[2,6-bis(1-methylethyl)phenyl]amino]carbonyl]amino]-2-phenylethyl]benzamide

(a) A mixture of 1.0 g (5.9 mmole) (±)-2-phenylglycinonitrile hydrochloride, 1.2 g (5.9 mmole) 2,6-diisopropylphenylisocyanate, 0.6 g (5.9 mmole) triethylamine and 10 mL $CH_2Cl_2$ was stirred at room temperature overnight after which the reaction mixture was washed with water, layers separated, and the organic solution was dried over $MgSO_4$. The solution was filtered and concentrated to dryness to give a yellow solid which was dissolved in ethyl acetate and heated with charcoal. The mixture was passed through celite and the filtrate concentrated in vacuo to give (±)-N-(cyanophenylmethyl)-N′-[2,6-bis(1-methylethyl)phenyl]urea as a white solid.

(b) The nitrile (3.0 g, 8.9 mmole) was dissolved in 100 mL of methanol with 0.5 mL of $H_2SO_4$ and hydrogenated using 0.1 g of 20% Pd/C. The methanol was concentrated to dryness and the product slurried in ethyl ether, filtered, and dried in vacuo at 40 °C for 2 hours to give N-(2-amino-1-phenylethyl)-N′-[2,6-bis(1-methylethyl)phenyl]urea, sulfate (1:1).

(c) To a 25-mL, three-neck flask equipped with magnetic stirrer were added 1.0 g (2.9 mmole) of the urea sulfate, 10 mL $CH_2Cl_2$ and 0.41 g (2.9 mmole) benzoyl chloride. To the mixture was added 5.9 g (5.9 mmole) triethylamine in one portion. The solution was stirred for 1 hour before forming a gelatinous precipitate. The solid was collected by filtration and the filtrate stripped to dryness. The combined solid was slurried in methanol, filtered, and washed with methanol. The product was dried in vacuo at 50 °C to give the title compound, m.p. 224-226 °C.

EXAMPLE 7

(±)-N-[2-[[[[2,6-bis(1-methylethyl)phenyl]amino]carbonyl]amino]-1-phenylethyl]heptanamide

(a) (±)-N-(Cyanophenylmethyl)heptanamide

(±)-2-Phenylglycinonitrile•HCl (2.0 g), THF (20 mL), and triethylamine (2.4 g) were added to a 50-mL flask fitted with stirring bar and drying tube. The triethylamine hydrochloride was removed by filtration and the filtrate was treated with heptanoic anhydride (2.88 g, 11.9 mmole) and stirred overnight. To the solution was added 10 mL of 1N NaOH. The solution was stirred for 10 minutes and then diluted with ethyl ether. The layers were separated and the organic portion was washed with water, 1N HCl, and saturated aqueous sodium chloride. The solution was dried over $MgSO_4$, filtered, and concentrated in vacuo to yield an orange-brown liquid. Trituration with hexane afforded a cream colored solid that was collected by filtration and washed with hexane to give compound (a) above.

(b) N-(2-Amino-1-phenylethyl)heptanamide

In 100 mL of methanolic ammonia 1.5 g of the heptanamide (a) was reduced using 1 g of Raney nickel under 50 psi. The solution was then stripped to dryness leaving a green liquid. The crude product (1.8 g) was used in the next step without further purification.

(c) (±)-N-[2-[[[[2,6-bis(1-methylethyl)phenyl]amino]carbonyl]amino-1-phenylethyl]heptanamide

To a 50-mL, one-neck flask were added the crude aminoamide (b) and 20 mL of ethyl acetate. In one portion 1.5 g (7.4 mmole) of 2,6-diisopropylphenylisocyanate was added causing a precipitate to form. The solid was collected by filtration and recrystallized from 50% ethyl acetate/50% hexane to give the title compound as a white solid, m.p. 187-189 °C.

Following the general procedure of Example 7 only substituting an appropriate amount of the acid anhydride or acid chloride of the acid listed below in Table 4 for heptanoic anhydride, the respective products listed below are obtained.

TABLE 4

| Acid | Product | m.p./°C |
|---|---|---|
| $CH_3COOH$ | (±)-N-[2-[[[[2,6-bis(1-methylethyl)phenyl]amino]carbonyl]amino]-1-phenylethyl]acetamide | 232-233 |
| $CH_3(CH_2)_8COOH$ | (±)-N-[2-[[[[2,6-bis(1-methylethyl)phenyl]amino]carbonyl]amino]-1-phenylethyl]decanamide | 204-205 |
| $CH_3(CH_2)_{10}COOH$ | (±)-N-[2-[[[[2,6-bis(1-methylethyl)phenyl]amino]carbonyl]amino]-1-phenylethyl]dodecanamide | 177-178 |
| $CH_3(CH_2)_{14}COOH$ | (±)-N-[2-[[[[2,6-bis(1-methylethyl)phenyl]amino]carbonyl]amino]-1-phenylethyl]hexadecanamide | 173-174 |
| $CH_3(CH_2)_{16}COOH$ | (±)-N-[2-[[[[2,6-bis(1-methylethyl)phenyl]amino]carbonyl]amino]-1-phenylethyl]octadecanamide | 168-170 |
| $(CH_3)_2C=CH-(CH_2)_2(CH_3)-CHCH_2COOH$ | (±)-N-[2-[[[[2,6-bis(1-methylethyl)phenyl]amino]carbonyl]amino]-1-phenylethyl]citronellamide | 189-191 |
| $CH_3(CH_2)_3COOH$ | (±)-N-[2-[[[[2,6-bis(1-methylethyl)phenyl]amino]carbonyl]amino]-1-phenylethyl]pentanamide | 223-225 |
| $CH_3(CH_2)_5COOH$ | (±)-N-[2-[[[[2,4-difluorophenyl]amino]carbonyl]amino]-1-phenylethyl]heptanamide* | MS $EI^+$ m/e = 404.5 |

* An appropriate amount of 2,4-difluorophenylisocyanate is substituted for 2,6-diisopropylphenylisocyanate.

EXAMPLE 8

(±)-N-[2,6-bis(1-methylethyl)phenyl]-N′-[2-heptylamino-2-phenylethyl]urea

To a 100-mL, one-neck flask equipped with stirring bar and $N_2$ inlet were added 20 mL of $CH_2Cl_2$ and 1.0 g (2.0 mmole) of (±)-N-[2-[[[[2,6-bis(1-methylethyl)phenyl]amino]carbonyl]amino]-1-phenylethyl]-heptanamide. To the solution was added a solution of Red-Al (3.4 M solution in toluene) in one portion (foaming). The mixture was stirred for 2 hours followed by the addition of more Red-Al due to the presence of starting material. The reaction was quenched with 5 mL of 2N NaOH. The layers were separated and the organic portion was washed once with saturated aqueous sodium chloride, the layers separated, and the solution was dried over $MgSO_4$, filtered, and removal of solvent in vacuo gave a colorless syrup. The material crystallized to give the title compound, m.p. 104-107 °C.

Following the general procedure of Example 8 only substituting an appropriate amount of the amide listed below in Table 5 for (±)-N-[2-[[[[2,6-bis(1-methylethyl)phenyl]amino]carbonyl]amino]-1-phenylethyl]-heptanamide the respective products listed in Table 5 are obtained.

TABLE 5

| Amide | Product | Physical Data |
|---|---|---|
| (±)-N-[2-[[[[2,6-bis(1-methylethyl)phenyl]-amino]carbonyl]amino]-1-phenylethyl]decanamide | (±)-N-[2,6-bis(1-methylethyl)-phenyl]-N'-2-decylamino-2-phenylethyl]urea | NMR (DMSO): δ 7.5 (br.s., 1H), 7.0-7.3 (m, 8H), 6.1 (br.s., 1H), 3.6 (br.s., 1H), 3.3 (2H), 3.1 (br.s., 4H), 2.3 (tr., 2H), 2.0 (br.s., 1H), 1.2 (s, 16H), 1.0 (d, 12H), 0.8 (tr., 3H) ppm. |
| (±)-N-[2-[[[[2,6-bis(1-methylethyl)phenyl]-amino]carbonyl]amino]-1-phenylethyl]dodecanamide | (±)-N-[2,6-bis(1-methylethyl)-phenyl]-N'-[2-dodecylamino-2-phenylethyl]urea | NMR (DMSO): δ 7.5 (br.s., 1H), 7.0-7.3 (m, 8H), 6.1 (br.s., 1H), 3.6 (br.s., 1H), 3.1 (m, 4H), 2.3 (m, 2H), 1.2 (s, 20H), 1.0 (d, 12H), 0.8 (tr., 3H) ppm. |
| (±)-N-[2-[[[[2,6-bis(1-methylethyl)phenyl]-amino]carbonyl]amino]-1-phenylethyl]hexadecan-amide | (±)-N-[2,6-bis(1-methylethyl)-phenyl-N'-[2-hexadecylamino-2-phenylethyl]urea | 80-82°C |
| (±)-N-[2-[[[[2,6-bis(1-methylethyl]phenyl]-amino]carbonyl]amino]-1-phenylethyl]octa-decanamide | (±)-N-[2,6-bis(1-methylethyl)-phenyl-N'-[2-octadecylamino-2-phenylethyl]urea | 84-86°C |

TABLE 5

| Amide | Product | Physical Data |
| --- | --- | --- |
| (±)-N-[2-[[[[2,6-bis(1-methylethyl)phenyl]-amino]carbonyl]amino]-1-phenylethyl]pentanamide | (±)-N-[2,6-bis(1-methylethyl)-phenyl]-N'-[2-pentylamino-2-phenylethyl]urea | 178-179°C |
| (±)-N-[2-[[[[2,4-di-fluorophenyl]amino]-carbonyl]amino]-1-phenylethyl]heptanamide | (±)-N-[2,4-difluorophenyl]-N'-[2-heptylamino-2-phenethyl]urea | |

EXAMPLE 9

(±)-N-[2-[[[[2,6-bis(1-methylethyl)phenyl]amino]carbonyl]amino]-1-phenylethyl]-6-bromohexanamide

To a 500-mL, three-neck flask equipped with addition funnel, $N_2$ inlet, drying tube, and magnetic stirrer were added 20.0 g (0.11 mole) (±)-2-phenylglycinonitrile•HCl, 250 mL TKF, and 22.2 g (0.22 mole) triethylamine. The mixture was stirred for 5 minutes, after which 23.5 g (0.11 mole) bromohexanoyl chloride was added dropwise over a period of 20 minutes.

The reaction proceeded for 1 hour. The insoluble material was removed by filtration and the solution was diluted with ethyl ether. The solution was washed with 1N HCl, saturated aqueous $NaHCO_3$, and saturated aqueous NaCl. The product was dried over $MgSO_4$, filtered, and concentrated to dryness in vacuo. The orange syrup was washed with hexane and stripped to dryness leaving a tan solid. The product was dried in vacuo at room temperature to give 6-bromo-N-(cyanophenylmethyl)hexanamide.

In 500 mL methanol, 24.98 g (0.081 mole) of the above obtained hexanamide, 4.5 mL sulfuric acid, and 1.0 g 20% Pd/C were combined and hydrogenated at 50 psi. The methanol was then concentrated to dryness leaving (±)-N-(2-amino-1-phenylethyl)-6-bromohexanamide sulfate (1:1) as a yellow foam (33.2 g) which was used in the next step without further purification.

To a solution of 500 mL ethyl acetate and 600 mL THF, was added 33.2 g (0.081 mole) of the amine salt followed by the addition of 16.4 g (0.081 mole) triethylamine in one portion. The mixture was stirred for 5 minutes, filtered, and 2,6-diisopropylphenyl isocyanate (16.4 g; 0.081 mole) was added to the filtrate in one portion with stirring. The reaction proceeded for 2 hours and the solvent was concentrated to dryness. The crude product was dissolved in ethyl acetate and it gradually crystallized on standing. The white solid was collected by filtration and dried in vacuo at 40° C for 3 hours to give the title compound, m.p. 176-178° C.

EXAMPLE 10

(±)-N-[2-[[[[2,6-bis(1-methylethyl)phenyl]amino]carbonyl]amino]-1-phenylethyl]-6-methoxyhexanamide

To a 25-mL, three-neck flask equipped with an overhead stirrer and reflux condenser were added 1 0 g (1.93 mmole) of (±)-N-[2-[[[[2,6-bis(1-methylethyl)phenyl]amino]carbonyl]amino]-1-phenylethyl]-6-bromohexanamide, 10 mL methanol, and 0.10 g (1.93 mmole) sodium methoxide. This mixture was heated to reflux for 24 hours. The solvent was removed in vacuo, leaving a white solid. The crude product was dissolved in 99% $CH_2Cl_2$/1% MeOH and passed through a silica gel column. Fractions containing only the product were combined and concentrated to dryness affording the title compound, m.p. 186-187° C.

EXAMPLE 11

(±)-N-[2,6-bis(1-methylethyl)phenyl]-N′-[3-dodecylamino-2-phenylpropyl]urea

To a suspension of 1.5 g (2.8 mmole) of N-[3-[[[[2,6-bis(1-methylethyl)phenyl]amino]carbonyl]amino]-2-phenylpropyl]dodecanamide in 50 mL toluene at room temperature under $N_2$ was added 3.5 mL Red-Al (3.4 M solution in toluene). Vigorous effervescence ensued. The resulting solution was heated to reflux for 5 hours. The solution was cooled and then quenched by the addition of 5 mL 1N NaOH and stirred overnight at room temperature. Water was added and the organic layer was separated, washed extensively with water, saturated aqueous NaCl, dried over $MgSO_4$, filtered, and concentrated in vacuo to give the title compound.
MS (EI) 522.06 m/e
IR (KBr) $\nu$max 3300, 2950, 1650, 1550, 1485, 1250, 700 $cm^{-1}$.
NMR ($CDCl_3$) $\delta$ 7.0-7.4 (m, 8H), 5.7 (br.s, 1.H), 4.8 (m, 1H), 3.5 (m, 2H), 3.2 (m, 2H), 2.9 (m, 2H), 2.4 (tr, 2H), 1.0-1.4 (m, 23H) ppm.

Following the general procedure of Example 11 only substituting an appropriate amount of N-[3-[[[[2,6-bis-(1-methylethyl)phenyl]amino]carbonyl]amino]-2-phenylpropyl]benzamide for N-[3-[[[[2,6-bis(1-methylethyl)phenylamino]amino]carbonyl]amino]-2-phenylpropyl]dodecanamide the following compound is

obtained:
(±)-N-[2,6-bis(1-methylethyl)phenyl]-N′-[3-benzylamino-2-phenylpropyl]urea, m.p. 135-138 °C.

EXAMPLE 12

(±)-N-[2-[[[[2,6-bis(1-methylethyl)phenyl]amino]carbonyl]amino]-1-phenethyl-2-pyridinylamide

To a THF suspension of 2-picolinic acid at room temperature under $N_2$ was added 1.1 g (0.0064 mole) carbonyldiimidazole and after 5 minutes complete dissolution occurred. A solution of the free amine of (±)-2-phenylglycinonitrile was prepared by adding 0.83 mL (0.0059 mole) of triethylamine to 1 g (0.0059 mole) of (±)-2-phenylglycinonitrile•HCl with stirring for 20 minutes followed by filtration. The filtrate was added to the previously prepared solution of 2-picolinic acid and carbonyldiimidazole and stirred at room temperature overnight. Water was added and the layers separated. The organic layer was washed with water, saturated aqueous NaCl, dried over $MgSO_4$, filtered, and concentrated in vacuo. The resulting N-(2-pyridinyl)-phenylglycinonitrile was recrystallized from ethyl acetate:hexane, affording 0.67 g of the nitrile which was reduced with Raney nickel in methanolic ammonia at 50 psi to give N-(2-amino-1-phenylethyl)-2-pyridinylamide. The pyridinylamide was used in the next step without further purification.

The pyridinylamide (0.8 g; 0.0033 mole) was dissolved in 25 mL $CH_2Cl_2$ at room temperature under $N_2$, after which 0.67 g (0.0033 mole) of 2,6-diisopropylphenylisocyanate was added. The solution was stirred at room temperature for 62 hours. Water was added and the organic layer was separated, washed with water, saturated aqueous NaCl, dried over $MgSO_4$, filtered, and concentrated in vacuo to give the title compound, m.p. 197-199 °C.

Following the general procedure of Example 12, only substituting an appropriate amount of 2-quinolylcarboxylic acid or 2-furanylcarboxylic acid for 2-picolinic acid, the following respective products were obtained:

(±)-N-[2-[[[[2,6-bis(1-methylethyl)phenyl]amino]carbonyl]amino]-1-phenethyl]-2-quinolylamide, m.p. 193-195 °C.

(±)-N-[2-[[[[2,6-bis(1-methylethyl)phenyl]amino]carbonyl]amino]-1-phenethyl]-2-furanylamide, m.p. 190-200 °C.

Following the general procedure of Example 12, only substituting an appropriate amount of 2-indolylcarboxylic acid for 2-picolinic acid and using as the coupling agent dicyclohexylcarbodiimide in methylene chloride, the following compound is obtained:

(±)-N-[2-[[[[2,6-bis(1-methylethyl)phenyl]amino]carbonyl]amino]-1-phenethyl]-2-indolylamide, m.p. >225 °C.

EXAMPLE 13

(±)-N-[2-[[[[2,6-bis(1-methylethyl)phenyl]amino]carbonyl]amino]-1-phenethyl]-2-quinolylamide, N-oxide

To a solution of 0.66 g (1.34 mmole) of (±)-N-[2-[[[[2,6-bis(1-methylethyl)phenyl]amino]carbonyl]-amino]-1-phenylethyl]-2-quinolylamide in 30 mL $CH_2Cl_2$ at room temperature under $N_2$ with stirring was added 0.31 g m-chloroperbenzoic acid (mCPBA). The solution was stirred at room temperature for 3 hours. An additional 0.31 g mCPBA was added and heated to reflux overnight after which an aqueous $NaHCO_3$ solution was added with stirring. The layers were separated and the organic layer was washed with water, saturated aqueous NaCl, over dried $MgSO_4$, filtered, and concentrated in vacuo to give the title compound, m.p. 176-181 °C.

EXAMPLE 14

S-(+)-N-[2-[[[[2,6-bis(1-methylethyl)phenyl]amino]carbonyl]amino]-1-phenylethyl]benzamide

(a) To a 1-L, three-neck round bottom flask were added 20.0 g (0.145 mole) (S)-(+)-phenylglycinol, 400 mL THF, 34.8 g (0.16 mole) butyloxycarbonyl (BOC) anhydride, and 17.7 g of 4-dimethylaminopyridine. The solution was stirred for 2 hours (under $N_2$) and monitored by TLC. The reaction mixture was stirred for an additional 2 hours, after which the solvent was stripped to dryness leaving a viscous liquid. The crude product was redissolved in ethyl acetate and washed with 2 x 75 mL KCl (1N), 1 x 75 mL NaOH (1N), and 1 x 75 mL of saturated aqueous NaCl. The solution was dried over $MgSO_4$, filtered, and

stripped to dryness. The white solid was slurried in hexane and collected by filtration to give the S-(+)-BOC phenylglycinol.

(b) This alcohol (17.2 g, 0.07 mole) was dissolved in 600 mL $CH_2Cl_2$ and cooled to 0° C. Triethylamine (14.1 g, 0.14 mole) was added in one portion followed by the dropwise addition of methanesulfonyl chloride (8.6 g, 0.077 mole). The mixture was vigorously stirred for 1 hour, after which 125 mL saturated aqueous NaCl was added and the layers separated. The organic layer was washed with an additional 175 mL saturated aqueous NaCl, and the organic phase dried with $MgSO_4$, filtered, and concentrated to dryness. The resulting solid was slurried in hexane and filtered to give the mesylate.

(c) The mesylate (15.0 g, 47.6 mmole) was dissolved in 180 mL DMF followed by the addition of 15.5 g (238 mmole) of sodium azide under an atmosphere of $N_2$. The mixture was heated to 80° C in a warm $H_2O$ bath for 1.5 hours. The mixture was cooled to room temperature followed by dilution with 180 mL of water. The product was extracted with two portions of $Et_2O$. The solvent was dried over $MgSO_4$, filtered, and concentrated to dryness. The liquid was chromatographed (silica gel; 25% ethyl acetate 75% hexane) affording the azide as a white solid.

(d) Lithium aluminum hydride (1.0 g, 25.7 mmole) was slurried in THF (90 mL) and cooled to -20° C followed by the addition of a solution of 5.0 g (19 mmole) of the above obtained azide in THF (40 mL). The mixture was slowly warmed to 15° C over 2 hours, quenched with aqueous $NaHSO_4$ at -35° C, followed by dilution with ethyl acetate. The mixture was filtered through celite and the solution was dried over $MgSO_4$, filtered, and concentrated, leaving (S)-(+)-β-butoxycarbonylamino-β-phenylethylamine as a colorless liquid.

(e) The thus obtained amine (4.5 g, 19 mmole) was dissolved in 130 mL ethyl acetate followed by the addition of 3.87 g (19 mmole) 2,6-diisopropylphenylisocyanate under an atmosphere of $N_2$. The precipitate formed immediately and was stirred for 3 hours. The product was collected by filtration, washed with hexane, and dried in vacuo.

(f) The resulting urea (6.3 g, 14.3 mmole) was slurried in 25 mL $CH_2Cl_2$ followed by the addition of HCl-(g) over 30 minutes at room temperature. The solution was concentrated to dryness, leaving a white solid which was dried in vacuo for 2 hours at room temperature to give (S)-(+)-N-[2,6-bis(1-methylethyl)-phenyl]-N′-[2-amino-2-phenylethyl)urea hydrochloride.

(g) The amine hydrochloride (1.3 g, 3.45 mmole) was slurried in 35 mL THF and 0.87 g (8.6 mmole) triethylamine. Benzoyl chloride (0.53 g, 3.6 mmole) was added in one portion and the reaction mixture was stirred overnight under $N_2$. The suspension was diluted with water and filtered. The white solid was dissolved in chloroform and washed with HCl (1N), NaOH (1N), and saturated aqueous NaCl then dried over $MgSO_4$, filtered, and concentrated to dryness, affording the title compound as a white solid.

(h) When in the foregoing procedure an appropriate amount of (R)-(-)-phenylglycinol is substituted for (S)-(+)-phenylglycinol the corresponding R(-)-N-[2-[[[[2,6-bis(1-methylethyl)phenyl]amino]carbonyl]-amino]-1-phenylethyl]benzamide is obtained.

EXAMPLE 15

S-(+)-N-[2,6-bis(1-methylethyl)phenyl]-N′-[2-(phenylmethylamino)-2-phenylethyl]urea

To a slurry of 1.65 g (3.17 mmole) of S-(+)-N-[2-[[[[2,6-bis(1-methylethy)phenyl]amino]carbonyl]amino]-1-phenylethyl]benzeneamide in 25 mL of toluene was added Red-Al (3.4 M solution in toluene) (4.4 mL diluted to 25 mL). The solution was refluxed for 1.5 hours and TLC indicated that more Red-Al was needed to complete the reaction. Additional Red-Al was added and the reaction mixture was stirred at reflux for 1.5 hours, after which the mixture was cooled in an ice bath and cautiously quenched with 40 mL NaOH (1N). The mixture was diluted with ethyl acetate and the layers were separated. The organic portion was dried over $MgSO_4$, filtered, and concentrated to dryness affording the title compound, m.p. 181-182° C.

When in the foregoing procedure an appropriate amount of R(-)-N-[2-[[[[2,6-bis(1-methylethyl)phenyl]-amino]carbonyl]amino]-1-phenylethyl]benzenamide is substituted for the S-(+)- compound the corresponding R(-)-N-[2,6-bis(1-methylethyl)phenyl]-N′-[2-(phenylmethylamino)-2-phenylethyl]urea is obtained.

EXAMPLE 16

(±)-N-[2,6-bis(1-methylethyl)phenyl]-N′-[2-[bis(phenylmethyl)amino]-2-phenylethyl]urea

1.5 g (3.5 mmole) of (±)-N-[2,6-bis(1-methylethyl)-phenyl]-N′-[2-phenylmethylamino)-2-phenylethyl]urea

was dissolved in 30 mL DMF followed by the addition of NaH (0.4 g, 0.015 mole). The reaction mixture was stirred for 5 minutes after which 0.63 g (3.7 mmole) benzyl bromide was added. The reaction mixture was stirred at room temperature for 30 minutes then diluted with water and extracted with $Et_2O$. The organic portion was dried over $MgSO_4$, filtered, and concentrated, leaving a viscous liquid. Chromatography (25% ethyl acetate/75% hexane) afforded the title compound as a colorless, viscous liquid. NMR ($CDCl_3$) : $\delta$ 7.07-7.4 (m, 19H), 4.6-4.8 (m, 1H), 32-3.6 (m, 7H), 2.8 (m, 2H), 0.9 (dd, 6H), 0.9 (dd, 6H) ppm.

Following the general procedure of Example 16, only substituting an appropriate amount of iodomethane for benzyl bromide, (±)-N-[2,6-bis(1-methylethyl)phenyl]-N′-[2-[methyl(phenylmethyl]amino]-2-phenylethyl]urea was obtained, m.p. 116-117 °C.

EXAMPLE 17

(±)-4-[6-[[2-[[[[2,6-bis(1-methylethyl)phenyl]amino]1-phenylethyl]amino]-6-oxohexyl]-1-piperazinecarboxylic acid, ethyl ester

Triethylamine (0.19 g), acetone (4 mL), and 0.16 g (3.8 mmole) N-carbethoxypiperazine were added to a 15-mL, one-neck flask with stirring, after which 1.0 g (1.9 mmole) of (±)-N-[2-[[[[2,6-bis(1-methylethyl)-phenyl]amino]carbonyl]amino]-1-phenylethyl]-6-bromohexanamide was added in one portion. The mixture was heated at reflux for 1 hour.

The solution was cooled to room temperature causing the formation of a white precipitate. The product was collected by filtration and washed with ethyl acetate.

The solid was dissolved in 50% THF/50% ethyl acetate and passed through silica gel. The solvent was stripped to dryness affording a white solid which was dried overnight in vacuo at 40 °C to give the title compound, m.p. 162-164 °C.

Following the general procedure of Example 17, only substituting an appropriate amount of ammonia, dimethylamine, or diethanolamine for carbethoxypiperazine, the following respective products are obtained:
(±)-N-[2-[[[[2,6-bis(1-methylethyl)phenyl]amino]carbonyl]amino]-1-phenylethyl]-6-aminohexanamideHBr salt, m.p. 188-190 °C;
(±)-N-[2-[[[[2,6-bis(1-methylethyl)phenyl] amino]carbonyl]amino]-1-phenylethyl]-6-dimethylaminohexanamide, m.p. 165-168 °C; and
(±)-N-[2-[[[[2,6-bis(1-methylethyl)phenyl]amino]carbonyl]amino]-1-phenylethyl]-6-diethanolaminohexanamide, m.p. 147-150 °C.

EXAMPLE 18

(S)-(+)-N-[2-(4-Dimethylaminobenzylamino)-2-phenethyl]-N′-[2,6-bis(1-methylethyl)phenyl]urea

(S)-(+)-N-(2-Amino-2-phenethyl)-N′-[2,6-bis(1-methylethyl)phenyl]urea•HCl (1.0 g, 2.6 mmole) was dissolved in 30 mL methanol followed by the addition of 1.0 g calcium sulfate and 0.3 g (2 mmole) of triethylamine. To this mixture 0.4 g (2.6 mmole) of 4-dimethylaminobenzaldehyde was added in one portion. The reaction mixture was stirred for 48 hours after which 0.5 g of sodium borohydride was added and stirring was continued for 10 minutes. The mixture was filtered, and the filtrate concentrated in vacuo leaving a foam which was dissolved in tetrahydrofuran and chromatographed on silica gel using tetrahydrofuran as the eluant. Fractions containing the product were concentrated to dryness, and the product was slurried in diethyl ether and concentrated in vacuo. The solid was triturated with hexane and collected by filtration to give the title compound, m.p. 160-161 °C.

EXAMPLE 19

N-[2,7-Bis(1-methylethyl)phenyl]-N′-[2-(phenylamino)2-phenylethyl)urea

A mixture of 1.5 g (14 mmole) of benzaldehyde, 35 mL methanol, 0.92 g (14.2 mmole) potassium cyanide, 1.47 g (15.8 mmole) aniline, and 2 mL of acetic acid was stirred at room temperature overnight. The mixture was concentrated in vacuo, and the remaining liquid was dissolved in ethyl acetate. This

mixture was washed with 25% aqueous sodium hydroxide, saturated aqueous sodium chloride, dried over $MgSO_4$, filtered, and concentrated to dryness. The yellow solid was slurried in hexane and collected by filtration to give N-phenyl-α-phenylaminoacetonitrile. The nitrile (1.75 g, 8.4 mmole) is hydrogenated with 1.5 g Raney nickel in 100 mL of methanolic ammonia at 50 p.s.i. The mixture was concentrated to dryness in vacuo leaving an oil which was triturated with diethyl ether. The insoluble material was removed by filtration, and the filtrate was dried in vacuo to give 2-phenylamino-2-phenylethylamine as an oil. To the amine (1.74 g, 8.2 mmole) dissolved in 50 mL of ethyl acetate was added 1.66 g (8.2 mmole) of 2,6-diisopropylphenylisocyanate in one portion. The mixture was stirred for 30 minutes at room temperature, and the solid was collected by filtration and washed with ethyl acetate and hexane to give the title compound.

EXAMPLE 20

(±)-N-[2-[[[[2,6-Bis(1-methylethyl)phenyl]amino]carbonyl]amino]-1-phenylethyl]-4-pyridinecarboxamide

Following the general procedure of Example 3 only substituting an appropriate amount of isonicotinoyl chloride for lauroyl chloride, the title compound was obtained, m.p. 172-174 °C.

EXAMPLE 21

Following the procedure of Example 6 only substituting an appropriate amount of 4-trifluoromethylbenzoyl chloride and 1-naphthoylchloride for benzoylchloride in Part 6(c) the following respective compounds were obtained:

(±)-N-[2-[[[[2,6-Bis(1-methylethyl)phenyl]amino]carbonyl]amino]-1-phenylethyl]-4-trifluoromethylbenzamide, m.p. 262-263 °C;

(±)-N-[2-[[[[2,6-Bis(1-methylethyl)phenyl]amino]carbonyl]amino]-1-phenylethyl]-1-naphthalenecarboxamide, m.p. 240-242 °C.

EXAMPLE 22

(±)-N-[2-[[[[2,6-Bis(1-methylethyl)phenyl]amino]carbonyl]amino]-1-phenylethyl]-2-pyrazinecarboxamide

Following the general procedure of Example 12 only substituting an appropriate amount of pyrazine-2-carboxylic acid for 2-picolinic acid, the title compound was obtained, m.p. 178-181 °C.

EXAMPLE 23

(±)-N-[3-[[[[2,6-Bis(1-methylethyl)phenyl]amino]carbonyl]amino]-2-phenylpropyl]-2-pyridinecarboxamide

Following the general procedure of Example 12 only substituting an appropriate amount of 2-cyano-2-phenylethylamine hydrochloride for (±)-2-phenylglycinonitrile, the title compound was obtained, m.p. 181-184 °C.

EXAMPLE 24

When in the procedure Example 13 appropriate amounts of the title compounds of Example 12, 22, and 23 are substituted for (±)-N-[2-[[[[2,6-bis(1-methylethyl)phenyl]amino]carbonyl]amino]-1-phenylethyl]-2-quinolinylamide, the following respective products were obtained:

(±)-N-[2-[[[[2,6-Bis(1-methylethyl)phenyl]amino]carbonyl]amino]-1-phenylethyl]-2-pyridinecarboxamide, 1-oxide, m.p. 105-110 °C;

(±)-N-[2-[[[[2,6-Bis(1-methylethyl)phenyl]amino]carbonyl]amino]-1-phenylethyl]-2-pyrazinecarboxamide, 1-oxide, m.p. 175-180 °C;

(±)-N-[3-[[[[2,6-Bis(1-methylethyl)phenyl]amino]carbonyl]amino]-2-phenylpropyl]-2-pyridinecarboxamide, 1-oxide, foam;

NMR ($CDCl_3$) : δ 8.34 (dd, 1H), 8.2 (d, 1H), 7.0-7.4 (m, 11H), 5.7 (s, 1H), 3.7 (tr, 2H), 3.4 (m, 2H), 3.05 (m, 2H), 1.1 (d, 12H) ppm.

* When in the procedure of Example 18 an appropriate amount of the aldehyde listed below was substituted for 4-dimethylaminobenzaldehyde and (±)-N-(2-amino-2-phenylethyl)-N'-[2,6-bis(1-methylethyl)-phenyl]urea•HCl (Example 25) or (R)-(-)-N-(2-amino-2-phenylethyl)-N'-[2,6-bis(1-methylethyl)phenyl]-urea•HCl (Examples 26-41) was substituted for the corresponding (S)-(+)- isomer, the products listed below were obtained:

| Example | Product | Aldehyde |
|---|---|---|
| 25 | (±)-N-[2,6-bis(1-methylethyl)phenyl]-N'-[2-[(2-pyridinylmethyl)amino]-2-phenylethylurea, m.p. 185-187°C. | Pyridine-2-carboxaldehyde |
| 26 | (R)-(-)-N-[2,6-bis(1-methylethyl)phenyl]-N'-[2-phenyl-2-[(2-thienyl-methyl)amino]ethyl]urea, m.p. 208-210°C. | Thiophen-2-carboxaldehyde |
| 27 | (R)-(-)-N-[2,6-bis(1-methylethyl)phenyl]-N'-[2-phenyl-2-[(3-pyridinylmethyl)amino]-ethyl]urea, m.p. 148-151°C. | Pyridine-3-carboxaldehyde |
| 28 | (R)-(-)-N-[2,6-bis(1-methylethyl)phenyl]N'-[2-phenyl-2-[(4-pyridinylmethyl)amino]-ethyl]urea, m.p. 196-198°C. | Pyridine-4-carboxaldehyde |

| Example | Product | Aldehyde |
|---|---|---|
| 29 | (R)-(-)-N-[2,6-bis(1-methylethyl)phenyl]-N'-[2-[(3-furanylmethyl)-amino]-2-phenylethyl]-urea, m.p. 175-176°C. | Furan-4-carboxaldehyde |
| 30 | (R)-(-)-N-[2,6-bis(1-methylethyl)phenyl]-N'-[2-[(cyclopropylmethyl)-amino]-2-phenylethyl]-urea, m.p. 188-190°C. | Cyclopropyl-carboxaldehyde |
| 31 | (R)-(-)-N-[2-(4-dimethyl-amino-benzylamino)-2-phenethyl]-N'-[2,6-bis(1-methylethyl)phenyl]urea, m.p. 162-163°C. | 4-Dimethylamino-benzaldehyde |
| 32 | (R)-(-)-N-[2,6-bis(1-methylethyl)phenyl]-N'-[2-[[(3-nitrophenyl)-methyl]amino]-2-phenylethyl]urea, m.p. 161-162°C. | 3-Nitrobenz-aldehyde |
| 33 | (R)-(-)-N-[2,6-bis(1-methylethyl)phenyl]-N'-[2-[[(2-hydroxyphenyl)-methyl]amino]-2-phenylethyl]urea, m.p. 157-158°C. | 2-Hydroxy-benzaldehyde |

| Example | Product | Aldehyde |
| --- | --- | --- |
| 34 | (R)-(-)-N-[2,6-bis(1-methylethyl)phenyl]-N'-[2-[[(4-methoxyphenyl)-methyl]amino]-2-phenylethyl]urea, m.p. 144-145°C. | 4-Methoxy-benzaldehyde |
| 35 | (R)-(-)-5-[[[2-[[[[2,6-bis(1-methylethyl)-phenyl]amino]carbonyl]-amino]-1-phenylethyl]-amino]methyl]-4-hydroxy-benzenesulfonic acid. NMR (DMSO): δ 7.5 (m, 6H), 7.2 (m, 2H), 7.1 (d, 2H), 6.8 (d, 1H), 6.2 (bs, 1H), 4.3 (bs, 1H), 3.7-3.5 (m, 5H), 3.4 (s, 1H), 3.0 (m, 2H), 1.1 (d, 12H) ppm. | o-benzaldehyde sulfonic acid |
| 36 | (R)-(-)-N-[2,6-bis(1-methylethyl)phenyl]-N'-[2-phenyl-2-[[[[2-(trifluoromethyl)phenyl]-methyl]amino]ethyl]urea, m.p. 176-178°C. | 2-Trifluoro-methylbenz-aldehyde |
| 37 | (R)-(-)-4-[[[2-[[[[2,6-bis(1-methylethyl)-phenyl]amino]carbonyl]-amino]-1-phenylethyl]-amino]methylbenzoic acid, m.p. 120-122°C. | 4-Carboxy-benzaldehyde |

| Example | Product | Aldehyde |
|---|---|---|
| 38 | (R)-(-)-N-[2,6-bis(1-methylethyl)phenyl]-N'-[2-[[(2-methoxyphenyl)-methyl]-amino]-2-phenylethyl]urea, m.p. 162-164°C. | 2-Methoxy-benzaldehyde |
| 39 | (R)-(-)-N-[2,6-bis(1-methylethyl)phenyl]-N'-[2-[[[3-[(dimethylamino)-methyl]-4-hydroxyphenyl]-methyl]amino]-2-phenyl-ethylurea. NMR ($CDCl_3$): δ 7.4-7.2 (m, 9H), 6.9 (d, 1H), 6.7 (d, 2H), 6.8 (bs, 1H), 4.8 (bs, 1H), 3.8 (m, 1H), 3.6 (t, 2H), 3.6-3.2 (m, 6H), 2.3 (s, 6H), 1.2 (d, 12H) ppm. | 3-Dimethylamino-methyl-4-hydroxy-benzaldehyde |
| 40 | (R)-(-)-N-[2,6-bis(1-methylethyl)phenyl]-N'-[2-[[(3,4-dihydroxy-phenyl)methyl]amino]-2-phenylethyl]urea. NMR ($CDCl_3$): δ 7.4-7.2 (m, 9H), 6.8 (d, 1H), 6.6 (t, 1H), 6.4 (d, 1H), 5.9 (bs, 1H), 4.3 (bs, 1H), 3.8-3.3 (m, 5H), 3.1 (bs, 2H), 1.1 (d, 12H) ppm. | 3,4-Dihydroxy-benzaldehyde |

| Example | Product | Aldehyde |
|---|---|---|
| 41 | (R)-(-)-N-[2,6-bis(1-methylethyl)phenyl]-N'-[2-[[(2,3-dihydroxyphenyl)methyl]amino]-2-phenylethyl]urea. NMR ($CDCl_3$): δ 7.4-7.2 (m, 9H), 6.8 (d, 1H), 6.6 (t, 1H), 6.4 (d, 1H), 5.9 (bs, 1H), 4.3 (bs, 1H), 3.8-3.3 (m, 5H), 3.1 (bs, 2H), 1.1 (d, 12H) ppm. | 2,3-Dihydroxybenzaldehyde |

EXAMPLE 42

(S)-(+)-N-[2,6-Bis(1-methylethyl)phenyl]-N'-[2-[[(4-methoxyphenyl)methyl]amino]-2-phenylethyl]urea

When in the procedure of Example 18 an appropriate amount of 4-methoxybenzaldehyde is substituted for 4-dimethylaminobenzaldehyde, the title compound was obtained, m.p. 144-145 °C.

EXAMPLE 43

(R)-(-)-N-[2,6-Bis(1-methylethyl)phenyl]-N'-[2-[[[4-(dimethylamino)phenyl]amino]-2-phenylethyl] urea hydrochloride

The title compound was obtained by treating the compound of Example 31 with hydrochloric acid.
NMR (DMSO) : δ 7.6-7.3 (m, 8H), 7.2-7.0 (m, 4H), 6.4 (bs, 1H), 4.3 (bs, 2H), 3.9-3.8 (m, 5H), 3.6 (m, 2H), 3.0 (s, 6H), 1.1 (d, 12H) ppm.

EXAMPLE 44

(R)-(-)-N-[2-[(Diphenylmethyl)amino]-1-phenylethyl]dodecanamide

(a) When in the procedure of Example 14(a) an appropriate amount of (R)-(-)-phenylglycinol is substituted for (S)-(-)-phenylglycinol and the general procedure of steps (a) through (d) are followed, (R)-(-)-β-butoxycarbonylamino-β-phenylethylamine was obtained.

(b) (R)-(β)-butoxycarbonylamino-β-phenylethylamine (10.0 g, 42.3 mmole) was dissolved in 250 mL of acetonitrile followed by the addition of 8.5 g (8.4 mmole) of triethylamine and 13 g (52.0 mmole) of benzhydrol bromide under a nitrogen atmosphere. The solution was refluxed for 2 hours after which the solvent was concentrated to dryness to give (R)-(-)-N-[β-(butoxycarbonylamino)-β-(phenyl)ethyl]-benzhydrylamine.

(c) The above obtained benzhydrylamine without further purification was dissolved in methylene chloride followed by the addition of HCl gas over a period of 30 minutes. The precipitate was collected by filtration, washed with hexane, and dried overnight in vacuo at 40 °C to give (R)-(-)-N-(2-amino-2-phenylethyl)benzhydrylamine hydrochloride.

(d) The benzhydrylamine salt obtained above (3.0 g, 7.9 mmole) was dissolved in 100 mL methanol

followed by the addition of 1.59 g of triethylamine. The solution was stirred for 30 minutes and concentrated in vacuo to give an oil which was triturated with 100 mL of tetrahydrofuran, precipitating triethylamine hydrochloride. The salt was removed by filtration and the filtrate was treated with 0.81 g of triethylamine and 1.75 g of dodecanoyl chloride. The slurry was stirred for 30 minutes, filtered, and concentrated to dryness. The crude product was slurried in 25% ethyl acetate/hexane, filtered hot, and chromatographed on silica gel. Fractions containing the title product were concentrated, and the oil obtained crystallized on standing.

NMR ($CDCl_3$) : δ 7.4-7.2 (m, 15H), 6.4 (d, 1H), 5.1 (q, 1H), 4.7 (s, 1H), 2.9 (m, 2H), 2.2 (t, 2H), 1.6 (bs, 3H), 1.3 (s, 16H), 0.9 (t, 3H) ppm.

EXAMPLE 45

(R)-(-)-N′-(Diphenylmethyl)-N-[(4-methoxyphenyl)methyl]-1-phenyl-1,2-ethanediamine

When in the general procedure of Example 44(d) an appropriate amount of 4-methoxybenzoyl chloride is substituted for dodecanoyl chloride, the title compound was obtained, m.p. 155-157 °C.

EXAMPLE 46

(R)-(-)-N′-(Diphenylmethyl)-N-[(4-methoxyphenyl)methyl]-1-phenyl-1,2-ethanediamine

The product of Example 45 (1.5 g, 3.4 mmole) was slurried in 20 mL toluene under a nitrogen atmosphere followed by the addition of Red-Al (5 mL of a 3.4 molar solution in toluene diluted with 5 mL toluene) in a steady stream. The solution was stirred at room temperature for overnight. The excess Red-Al was cautiously quenched with sodium hydroxide (1N, 10 mL) followed by dilution with chloroform. The layers were separated, and the organic portion was dried over magnesium sulfate, filtered, and concentrated to dryness. The crude product was dissolved in 25% ethyl acetate/75% hexane and passed through a bed of silica gel. Fractions containing the product were combined and dried in vacuo to give the title compound as a colorless viscous liquid.

($CDCl_3$) : δ 7.4-7.2 (m, 17H), 6.8 (d, 2H), 4.7 (s, 1H), 3.8 (s, 3H), 3.7-3.4 (m, 4H), 2.7 (m, 1H), 2.0 (bs, 1H) ppm.

Formula Chart

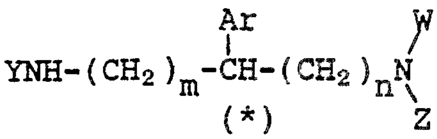 (Formula I)

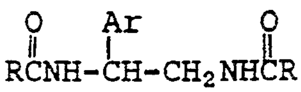 (28)

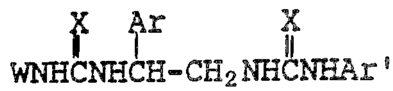 (27)

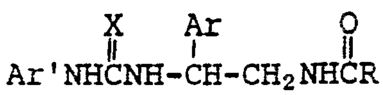 (29)

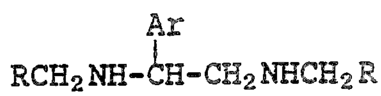 (31)

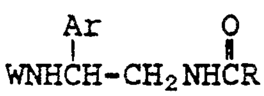 (30)

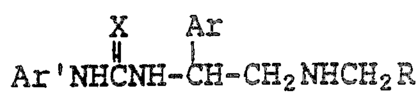 (32)

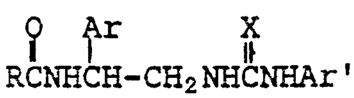 (25)

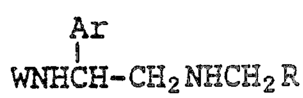 (33)

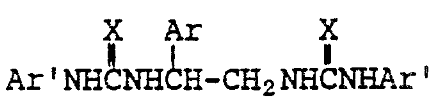 (26)

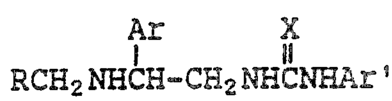 (34)

CHART I

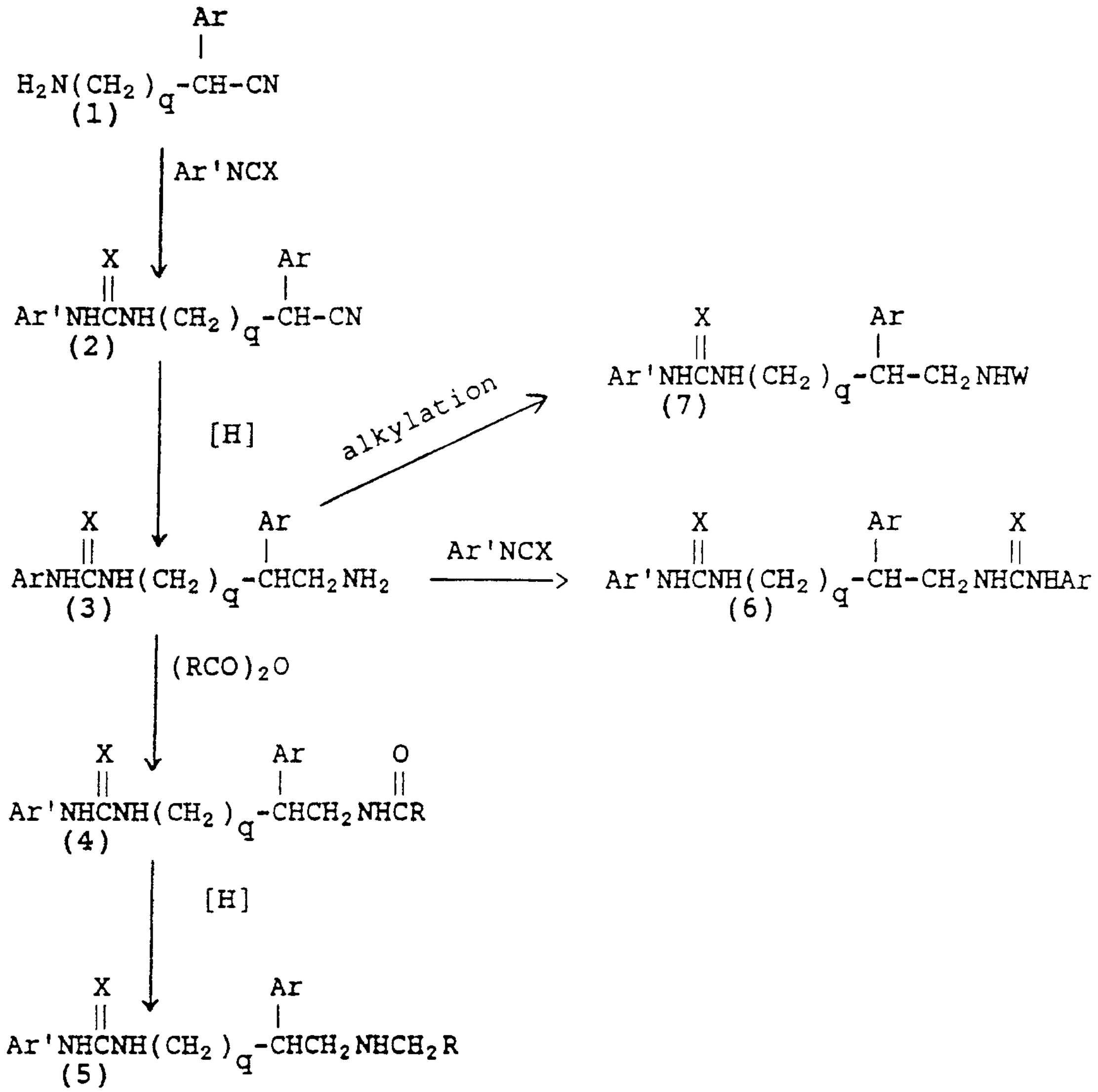


(1) (2) (3) (4) (5) (6) (7)

CHART II

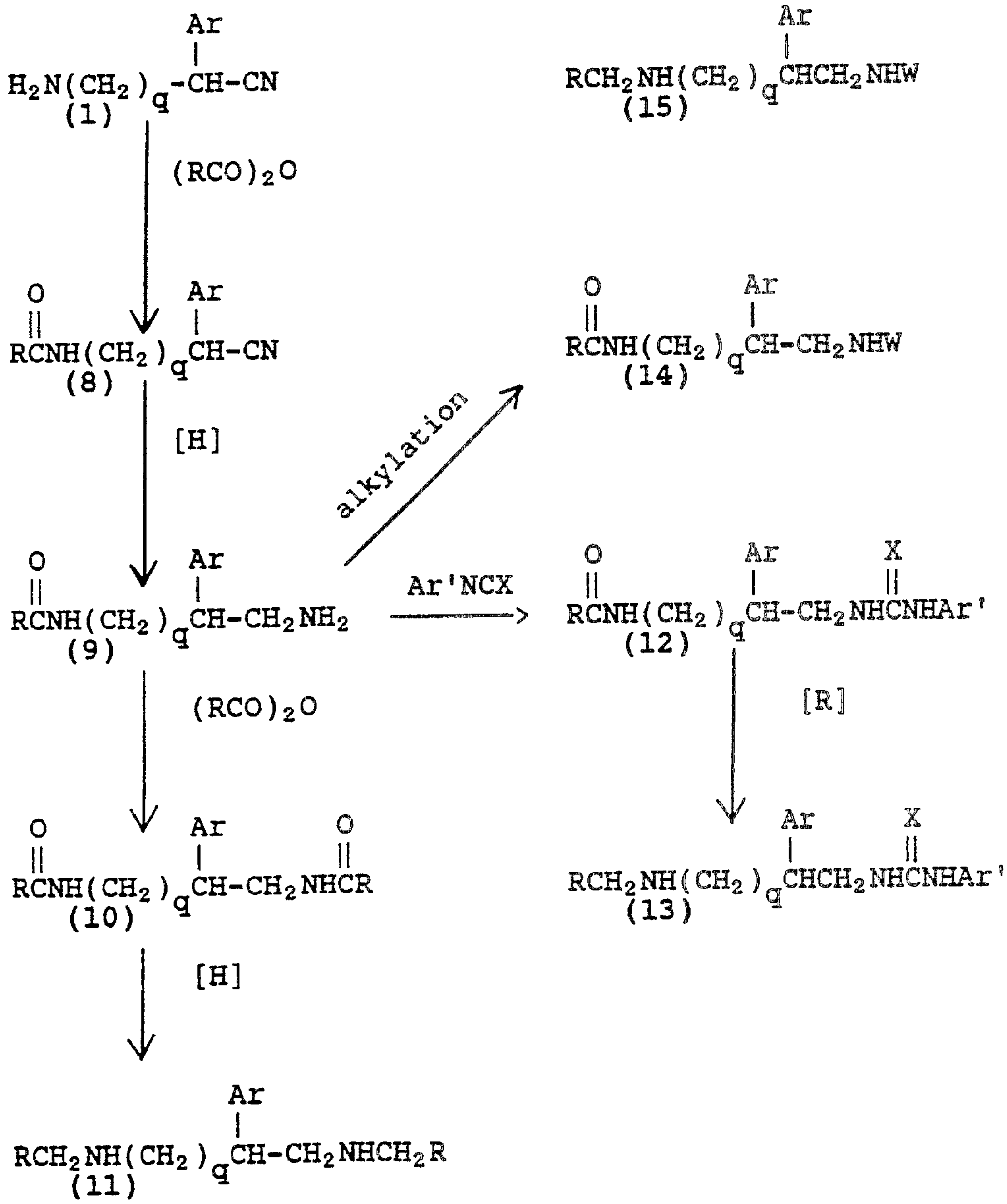


(1) (8) (9) (10) (11) (15) (14) (12) (13)

CHART III

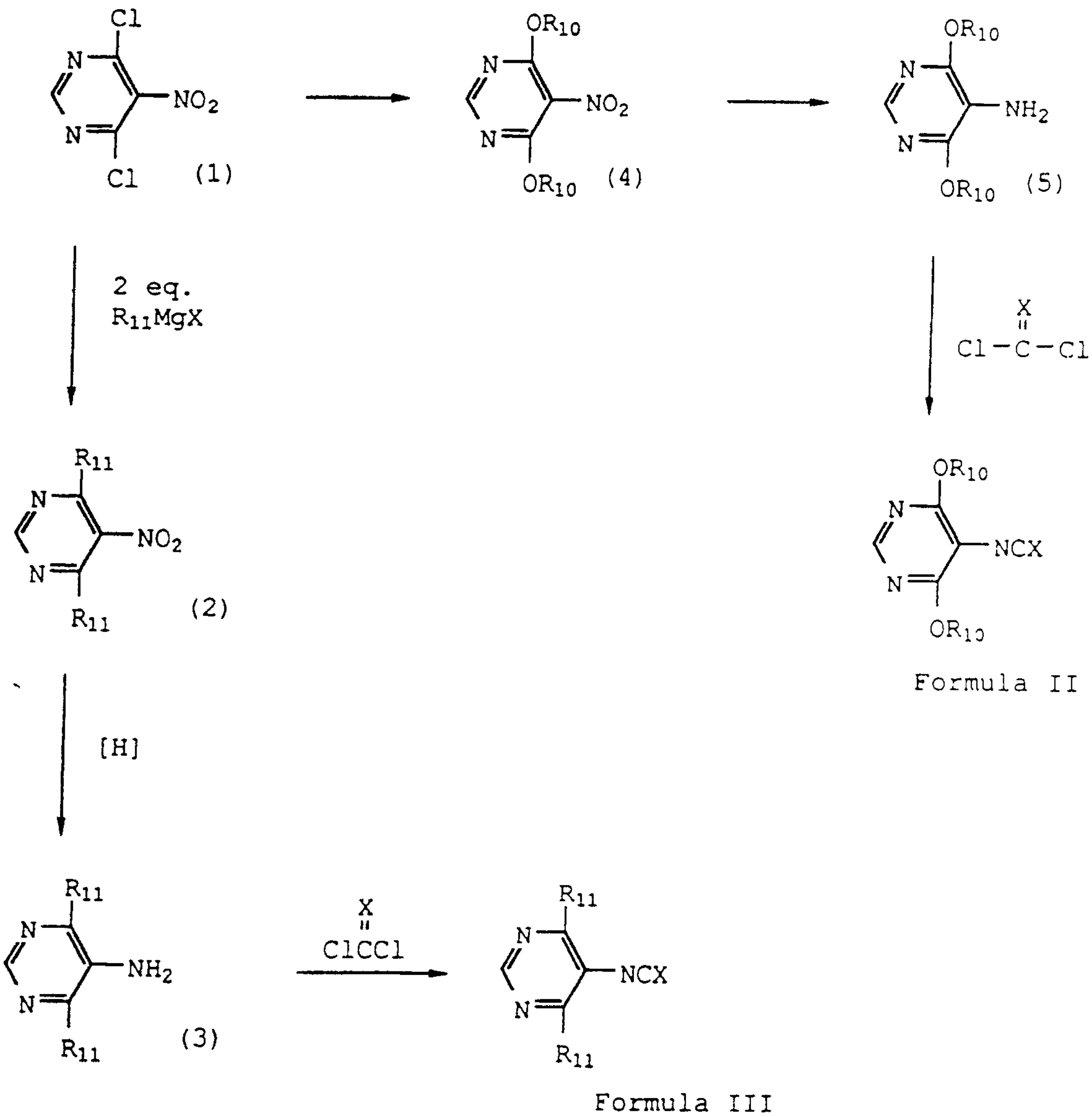

CHART IV

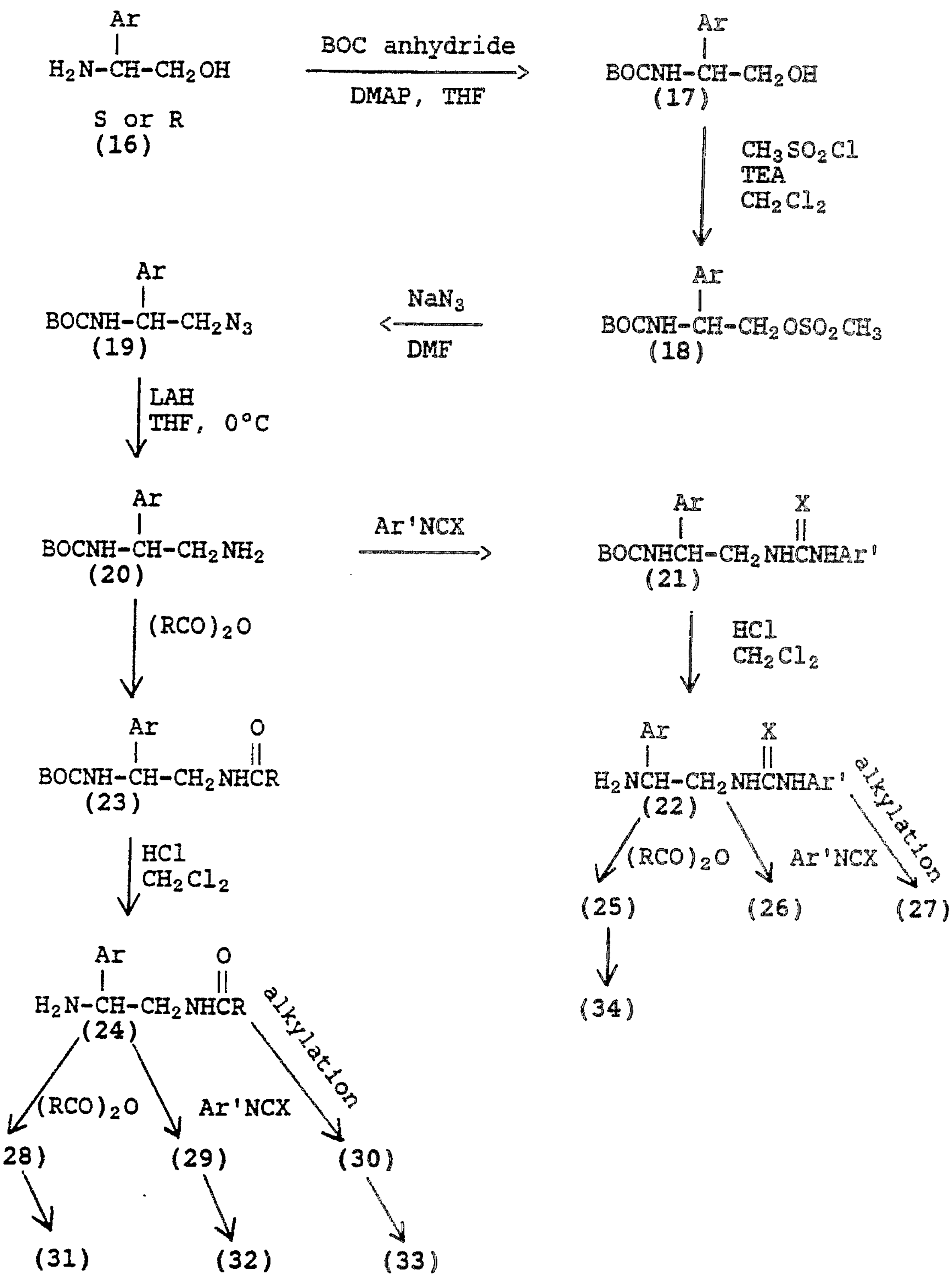


## Claims

1. A compound of the formula

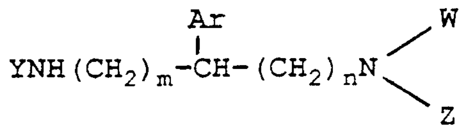

wherein each of m and n is zero or one;
wherein Ar is
(a) phenyl which is unsubstituted or is substituted with from 1 to 3 substituents selected from
alkyl having from 1 to 6 carbon atoms and which is straight or branched,
alkoxy having from 1 to 6 carbon atoms and which is straight or branched;
phenoxy,
hydroxy,
fluorine,
chlorine,
bromine,
nitro,
trifluoromethyl,
-COOH,
-COOalkyl wherein alkyl has from 1 to 4 carbon atoms,
$-NR_1R_2$ wherein
$R_1$ and $R_2$ are independently hydrogen or alkyl of from 1 to 4 carbon atoms;
(b) 1- or 2-naphthyl which is unsubstituted or substituted with
alkyl having from 1 to 6 carbon atoms and which is straight or branched;
alkoxy having from 1 to 6 carbon atoms and which is straight or branched,
hydroxy,
fluorine,
chlorine,
bromine,
nitro,
trifluoromethyl,
-COOH,
-COOalkyl wherein alkyl has from 1 to 4 carbon atoms,
$-NR_1R_2$ wherein $R_1$ and $R_2$ are as defined above; or
(c) a 5- or 6-membered monocyclic or fused bicyclic heterocycle containing at least 1 to 4 nitrogen, oxygen or sulfur atoms in at least one ring member;
wherein Y and Z are independently selected from:
(a) hydrogen;

(b) 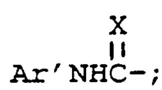

(c) 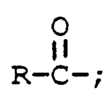

(d) $R-CH_2$; and

(e) 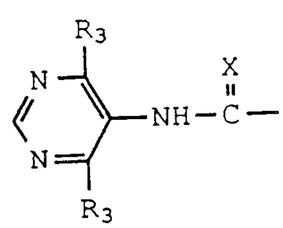

wherein $R_3$ is straight or branched alkyl having from 1 to 4 carbon atoms or straight branched alkoxy having from 1 to 4 carbon atoms;

wherein X is oxygen or sulfur;

wherein Ar′ is selected from:

(a) phenyl which is unsubstituted or is substituted with from 1 to 3 substituents selected from
alkyl having from 1 to 6 carbon atoms and which is straight or branched,
alkoxy having from 1 to 6 carbon atoms and which is straight or branched,
phenoxy,
hydroxy,
fluorine,
chlorine,
bromine,
nitro,
trifluoromethyl,
-COOH,
-COOalkyl wherein alkyl has from 1 to 4 carbon atoms,
$-NR_1R_2$ wherein
$R_1$ and $R_2$ are independently hydrogen or alkyl of from 1 to 4 carbon atoms; and

(b) 1- or 2-naphthyl which is unsubstituted or substituted with
alkyl having from 1 to 6 carbon atoms and which is straight or branched,
alkoxy having from 1 to 6 carbon atoms and which is straight or branched,
hydroxy,
fluorine,
chlorine,
bromine,
nitro,
trifluoromethyl,
-COOH,
-COOalkyl wherein alkyl has from 1 to 4 carbon atoms,
$-NR_1R_2$ wherein $R_1$ and $R_2$ are as defined above;

wherein R is selected from:

(a) a straight or branched hydrocarbon chain having from 1 to 20 carbon atoms and which is saturated or contains from 1 to 3 double bonds;

(b) a straight or branched hydrocarbon chain having from 1 to 20 carbon atoms wherein the terminal carbon atom is substituted with chlorine; fluorine; bromine; straight or branched lower alkoxy having from 1 to 4 carbon atoms; straight or branched thioalkoxy having from 1 to 4 carbon atoms; a $-COOR_4$ group wherein $R_4$ is hydrogen or a straight or branched alkyl having from 1 to 4 carbon atoms; an $-NR_5R_6$ group wherein $R_5$ and $R_6$ are independently hydrogen or lower alkyl having from 1 to 4 carbon atoms wherein said alkyl is unsubstituted or is substituted with hydroxy, or wherein $-NR_5R_6$ taken together form a monocyclic heterocyclic group selected from pyrrolidino, piperidino, piperazino, or piperazino substituted in the 4-position with a lower alkyl having from 1 to 4 carbon atoms or $-COOR_4$ wherein $R_4$ has the meaning defined above; and

(c) A 5- or 6-membered monocyclic or fused bicyclic heterocycle containing at least 1 to 4 nitrogen, oxygen or sulfur atoms in at least one ring member;

(d) the group

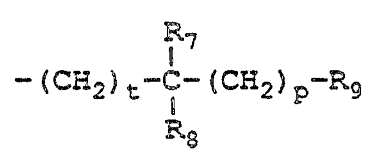

wherein t is zero to 4; p is zero to 4 with the proviso that the sum of t and p is not greater than 5; $R_7$ and $R_8$ are independently selected from hydrogen or straight or branched alkyl having from 1 to 6 carbon atoms, or when $R_7$ is hydrogen, $R_8$ can be selected from the groups defined for $R_9$; and $R_9$ is phenyl or phenyl substituted with from 1 to 3 substituents selected from straight or branched alkyl having from 1 to 6 carbon atoms, straight or branched alkoxy having from 1 to 6 carbon atoms, straight or branched thioalkoxy having from 1 to 6 carbon atoms, phenoxy, hydroxy, fluorine, chlorine, bromine, nitro, trifluoromethyl,

-COOH, COOalkyl wherein alkyl has from 1 to 4 carbon atoms, or $NR_5R_6$ wherein $R_5$ and $R_6$ having the meanings defined above;

(e) phenyl or phenyl substituted with from 1 to 3 substituents selected from straight or branched alkyl having from 1 to 6 carbon atoms, straight or branched alkoxy having from 1 to 6 carbon atoms, straight or branched thioalkoxy having from 1 to 6 carbon atoms, phenoxy, hydroxy, fluorine, chlorine, bromine, nitro, trifluoromethyl, -COOH, COOalkyl wherein alkyl has from 1 to 4 carbon atoms, or $-NR_5R_6$ wherein $R_5$ and $R_6$ have the meanings defined above; and

(f) 1- or 2-naphthyl or 1- or 2-naphthyl substituted with from 1 to 3 substituents selected from straight or branched alkyl having from 1 to 6 carbon atoms, straight or branched alkoxy having from 1 to 6 carbon atoms, hydroxy, chlorine, fluorine, bromine, nitro, trifluoromethyl, -COOH, COOalkyl wherein alkyl has from 1 to 4 carbon atoms, or $-NR_5R_6$ wherein $R_5$ and $R_6$ have the meanings defined above;

wherein W is selected from:

(a) hydrogen;

(b) a straight or branched hydrocarbon chain having from 1 to 20 carbon atoms and which is saturated or contains from 1 to 3 double bonds;

(c) a straight or branched hydrocarbon chain having from 1 to 20 carbon atoms wherein the terminal carbon atom is substituted with chlorine; fluorine; bromine; straight or branched lower alkoxy having from 1 to 4 carbon atoms; straight or branched thioalkoxy having from 1 to 4 carbon atoms; a $-COOR_4$ group wherein $R_4$ has the meaning defined above; an $-NR_5R_6$ group wherein $R_5$ and $R_6$ have the meaning defined above;

(d) the group

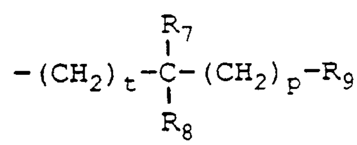

wherein t, p, $R_7$, $R_8$, and $R_9$ have the meanings defined above;

(e) phenyl or phenyl substituted with from 1 to 3 substituents selected from straight or branched alkyl having from 1 to 6 carbon atoms, straight or branched alkoxy having from 1 to 4 carbon atoms, straight or branched thioalkoxy having from 1 to 4 carbon atoms, phenoxy, hydroxy, $SO_3H$, fluorine, chlorine, bromine, nitro, trifluoromethyl, -COOH, COOalkyl wherein alkyl has from 1 to 4 carbon atoms, or $-(CH_2)_s-NR_5R_6$ wherein s is zero to 2, and $R_5$ and $R_6$ have the meanings defined above; and

(f) the group

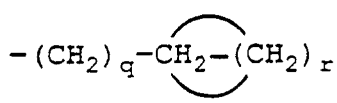

wherein q is zero to 2 and r is 2 to 6; or a pharmaceutically acceptable salt and N-oxides thereof; with the provisos:

(a) m and n are not zero at the same time;

(b) where both Z and W are the group

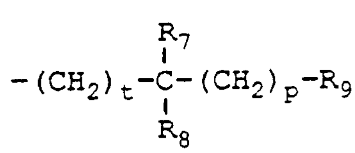

$R_8$ and $R_9$ are not the same;

(c) each of Y, Z, and W are not hydrogen at the same time; and

(d) when Ar represents a 5- or 6-membered monocyclic or fused bicyclic heterocycle, one of m or n is zero.

2. A compound of Claim 1 wherein one of W and Z is hydrogen.

3. A compound of Claim 2 wherein Y is selected from:

(a) 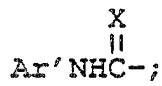

(b)

R-

(b) 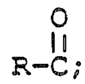

and

(c) $RCH_2$-.

4. A compound of Claim 3 wherein Y is

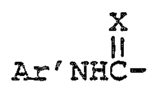

and X is oxygen.

5. A compound of Claim 4 wherein Ar′ is 2,6-disubstituted.

6. A compound of Claim 5 wherein Z is

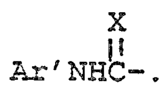

7. A compound of Claim 6 wherein X is oxygen.

8. A compound of Claim 1 which is N′,N‴-[2,6-bis(1-methylethyl)phenyl]-N,N″-(2-phenyl-1,3-propanediyl)bis-urea.

9. A compound of Claim 5 wherein Z is

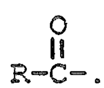

10. A compound of Claim 9 which is selected from:
N-[3-[[[[2,6-bis(1-methylethyl)phenyl]amino]carbonyl]amino]-2-phenylpropyl]heptanamide,
N-[3-[[[[2,6-bis(1-methylethyl)phenyl]amino]carbonyl]amino]-2-phenylpropyl]dodecanamide,
(±)-N-[2-[[[[2,6-bis(1-methylethyl)phenyl]amino]carbonyl]amino]-1-phenylethyl]benzenecarboxamide,
(±)-N-[2-[[[[2,6-bis(1-methylethyl)phenyl]amino]carbonyl]amino]-2-phenylethyl]benzamide,
(±)-N-[2-[[[[2,6-bis(1-methylethyl)phenyl]amino]carbonyl]amino]-1-phenylethyl]heptanamide,
(±)-N-[2-[[[[2,6-bis(1-methylethyl)phenyl]amino]carbonyl]amino]-1-phenylethyl]-6-bromohexanamide,
(±)-N-[2-[[[[2,6-bis(1-methylethyl)phenyl]amino]carbonyl]amino]-1-phenylethyl]-6-methoxyhexanamide,
(±)-N-[2-[[[[2,6-bis(1-methylethyl)phenyl]amino]carbonyl]amino]-1-phenylethyl]-2-pyridinylamide,
(±)-N-[2-[[[[2,6-bis(1-methylethyl)phenyl]amino]carbonyl]amino]-1-phenylethyl]-2-quinolylamide, N-oxide,
(±)-N-[2-[[[[2,6-bis(1-methylethyl)phenyl]amino]carbonyl]amino]-1-phenylethyl]benzamide,
(±)-N-[2-[[[[2,6-bis(1-methylethyl)phenyl]amino]carbonyl]amino]-1-phenylethyl]-2,6-dichlorobenzenecarboxamide,
(±)-N-[2-[[[[2,6-bis(1-methylethyl)phenyl]amino]carbonyl]amino]-1-phenylethyl]-4-methylbenzenecarboxamide,
(±)-N-[2-[[[[2,6-bis(1-methylethyl)phenyl]amino]carbonyl]amino]-1-phenylethyl]-4-methoxybenzenecarboxamide,
(±)-N-[2-[[[[2,6-bis(1-methylethyl)phenyl]amino]carbonyl]amino]-1-phenylethyl]acetamide,
(±)-N-[2-[[[[2,6-bis(1-methylethyl)phenyl]amino]carbonyl]amino]-1-phenylethyl]decanamide,
(±)-N-[2-[[[[2,6-bis(1-methylethyl)phenyl]amino]carbonyl]amino]-1-phenylethyl]dodecanamide,
(±)-N-[2-[[[[2,6-bis(1-methylethyl)phenyl]amino]carbonyl]amino]-1-phenylethyl]hexadecanamide,
(±)-N-[2-[[[[2,6-bis(1-methylethyl)phenyl]amino]carbonyl]amino]-1-phenylethyl]octadecanamide,
(±)-N-[2-[[[[2,6-bis(1-methylethyl)phenyl]amino]carbonyl]amino]-1-phenylethyl]citronellamide,
(±)-N-[2-[[[[2,6-bis(1-methylethyl)phenyl]amino]carbonyl]amino]-1-phenylethyl]pentanamide,

(±)-N-[2-[[[[2,6-bis(1-methylethyl)phenyl]amino]carbonyl]amino]-1-phenylethyl]heptanamide,
(±)-N-[2-[[[[2,6-bis(1-methylethyl)phenyl]amino]carbonyl]amino]-1-phenylethyl]-6-aminohexanamide, HBr salt,
(±)-N-[2-[[[[2,6-bis(1-methylethyl)phenyl]amino]carbonyl]amino]-1-phenylethyl]-6-dimethylaminohexanamide,
(±)-N-[2-[[[[2,6-bis(1-methylethyl)phenyl]amino]carbonyl]amino]-1-phenylethyl]-2-indolylamide,
(±)-N-[2-[[[[2,6-bis(1-methylethyl)phenyl]amino]carbonyl]amino]-1-phenylethyl]-2-furanylamide,
(±)-6-[2-[[[[2,6-bis(1-methylethyl)phenyl]amino]carbonyl]amino]-1-phenylethyl]amino]carbonyl]hexanoic acid methyl ester,
N-[3-[[[[2,6-bis(1-methylethyl)phenyl]amino]carbonyl]amino]-2-phenylpropyl]benzamide,
N-[3-[[[[2,6-bis(1-methylethyl)phenyl]amino]carbonyl]amino]-2-phenylpropyl]pentanamide,
N-[3-[[[[2,6-bis(1-methylethyl)phenyl]amino]carbonyl]amino]-2-(m-hydroxyphenyl)propyl]benzamide,
(R)-(-)-N-[2-[[[[2,6-bis(methylethyl)phenyl]amino]carbonyl]amino]-1-phenylethyl]benzamide,
(±)-N-[2-[[[[2,6-bis(1-methylethyl)phenyl]amino]carbonyl]amino]-1-phenylethyl]-4-trifluoromethylbenzamide,
(±)-N-[2-[[[[2,6-bis(1-methylethyl)phenyl]amino]carbonyl]amino]-1-phenylethyl]-1-naphthalenecarboxamide,
(±)-N-[2-[[[[2,6-bis(1-methylethyl)phenyl]amino]carbonyl]amino]-1-phenylethyl]-2-pyrazinecarboxamide,
(±)-N-[3-[[[[2,6-bis(1-methylethyl)phenyl]amino]carbonyl]amino]-2-phenylpropyl]-2-pyridinecarboxamide,
(±)-N-[2-[[[[2,6-bis(1-methylethyl)phenyl]amino]carbonyl]amino]-1-phenylethyl]-2-pyridinecarboxamide, 1-oxide,
(±)-N-[2-[[[[2,6-bis(1-methylethyl)phenyl]amino]carbonyl]amino]-1-phenylethyl]-2-pyrazinecarboxamide, 1-oxide,
(±)-N-[3-[[[[2,6-bis(1-methylethyl)phenyl]amino]carbonyl]amino]-1-phenylethyl]-2-phenylpropyl]-2-pyridinecarboxamide, 1-oxide,
(±)-N-[2-[[[[2,6-bis(1-methylethyl)phenyl]amino]carbonyl]amino]-1-phenylethyl]-4-pyridinecarboxamide,
(R)-(-)-N-[2-[(diphenylmethyl)amino]-1-phenyl-ethyl]dodecanamide, and
(±)-N-[2-[[[[2,6-bis(1-methylethyl)phenyl]amino]carbonyl]amino]-1-phenylethyl]-2-quinolylamide.

11. A compound of Claim 5 wherein Z is $RCH_2$.

12. A compound of Claim 11 which is selected from:
(±)-N-[2,6-bis(1-methylethyl)phenyl]-N'-[3-dodecyclamino-2-phenylpropyl]urea,
S(+)-N-[2,6-bis(1-methylethyl)phenyl]-N'-[2-(phenylmethylamino)-2-phenylethyl]urea,
(±)-N-[2,6-bis(1-methylethyl)phenyl]-N'-[2-[bis(phenylmethyl)amino]-2-phenylethyl]urea,
N-[2-(4-dimethylaminobenzylamino)-2-phenethyl]-N'-[2,6-bis(1-methylethyl)phenyl]urea,
(±)-N-[2,6-bis(1-methylethyl)phenyl]-N'-[2-phenylmethylamino]-2-phenylethyl]urea and the hydrochloride salt,
(±)-N-[2,6-bis(1-methylethyl)phenyl]-N'-[2-heptylamino-2-phenylethyl]urea,
(±)-N-[2,6-bis(1-methylethyl)phenyl]-N'-[2-decylamino-2-phenylethyl]urea,
(±)-N-[2,6-bis(1-methylethyl)phenyl]-N'-[2-dodecylamino-2-phenylethyl]urea,
(±)-N-[2,6-bis(1-methylethyl)phenyl]-N'-[2-octadecylamino-2-phenylethyl]urea,
(±)-N-[2,6-bis(1-methylethyl)phenyl]-N'-[2-hexadecylamino-2-phenylethyl]urea,
(±)-N-[2,6-bis(1-methylethyl)phenyl]-N'-[2-pentylamino-2-phenylethyl]urea,
(±)-N-[2,4-difluorophenyl]-N'-[2-heptylamino-2-phenylethyl]urea,
(±)-N-[2,6-bis(1-methylethyl)phenyl]-N'-[3-benzylamino-2-phenylpropyl]urea,
(±)-N-[2,6-bis(1-methylethyl)phenyl]-N'-[2-(phenylmethylamino)-1-phenylethyl]urea,
(±)-N-[2,6-bis(1-methylethyl)phenyl]-N'-[2-(6-methoxyhexylamino)-1-phenylethyl]urea,
(±)-N-[2,6-bis(1-methylethyl)phenyl]-N'-[2-[(2-pyridinylmethyl)amino]-2-phenylethyl]urea,
(R)-(-)-N-[2,6-bis(1-methylethyl)phenyl]-N'-[2-phenyl-2-[(2-thienylmethyl)amino]ethyl]urea,
(R)-(-)-N-[2,6-bis(1-methylethyl)phenyl]-N'-[2-phenyl-2-[(3-pyridinylmethyl)amino]ethyl]urea,
(R)-(-)-N-[2,6-bis(1-methylethyl)phenyl]-N'-[2-phenyl-2-[(4-pyridinylmethyl)amino]ethyl]urea,
(R)-(-)-N-[2,6-bis(1-methylethyl)phenyl]-N'-[2-phenyl-2-[(3-furanylmethyl)amino]-2-phenylethyl]urea,
(R)-(-)-N-[2,6-bis(1-methylethyl)phenyl]-N'-[2-[(cyclopropylmethyl)amino]-2-phenylethyl]urea,
(R)-(-)-N-[2-(4-dimethylaminobenzylamino)-2-phenethyl]-N'-[2,6-bis(1-methylethyl)phenyl]urea,
(R)-(-)-N-[2,6-bis(1-methylethyl)phenyl]-N'-[2-[[(3-nitrophenyl)methyl]amino]-2-phenylethyl]urea,
(R)-(-)-N-[2,6-bis(1-methylethyl)phenyl]-N'-[2-[[(2-hydroxyphenyl)methyl]amino]-2-phenylethyl]urea,
(R)-(-)-N-[2,6-bis(1-methylethyl)phenyl]-N'-[2-[[(4-methoxyphenyl)methyl]amino]-2-phenylethyl]urea,
(R)-(-)-5-[[[2-[[[[2,6-bis(1-methylethyl)phenyl]amino]carbonyl]amino]-1-phenylethyl]amino]methyl]-4-hydroxybenzenesulfonic acid,
(R)-(-)-N-[2,6-bis(1-methylethyl)phenyl]-N'-[2-phenyl-2-[[[2-(trifluoromethyl)phenyl]methyl]amino]ethyl]urea,
(R)-(-)-4-[[[2-[[[[2,6-bis(1-methylethyl)phenyl]amino]carbonyl]amino]-1-phenylethyl]amino]methylbenzoic acid,
(R)-(-)-N-[2,6-bis(1-methylethyl)phenyl]-N'-[2-phenyl-2-[[[2-methoxyphenyl)methyl]amino]-2-phenylethyl]urea,
(R)-(-)-N-[2,6-bis(1-methylethyl)phenyl]-N'-[2-phenyl-2-[[[3-[(dimethylamino)methyl]-4-hydroxyphenyl]methyl]-amino]-2-phenylethylurea,
(R)-(-)-N-[2,6-bis(1-methylethyl)phenyl]-N'-[2-phenyl-2-[[(3,4-dihydroxyphenyl)methyl]amino]2-phenylethyl]urea,
(R)-(-)-N-[2,6-bis(1-methylethyl)phenyl]-N'-[2-[[(2,3-dihydroxyphenyl)methyl]amino]-2-phenylethyl]urea,

(S)-(+)-N-[2,6-bis(1-methylethyl)phenyl]-N′-[2-[[(4-methoxyphenyl)methyl]amino]-2-phenylethyl]urea, (R)-(-)-N-[2,6-bis(1-methylethyl)phenyl]-N′-[2-[[[4-(dimethylamino)phenyl]methyl]amino]-2-phenylethyl]urea hydrochloride,
(R)-(-)-N′-(diphenylmethyl)-N-[(4-methoxyphenyl)methyl]-1-phenyl-1,3-ethanediamine, and
(R)-(-)-N′-(diphenylmethyl)-N-[(4-methoxyphenyl)methyl]-1-phenyl-1,2-ethanediamine.

13. A pharmaceutical composition comprising a compound of Claim 1 in a pharmaceutically acceptable carrier.

14. Use of a compound as defined in Claims 1 to 12 for the preparation of a pharmaceutical composition useful for controlling the blood cholesterol.

15. A process for preparing a compound as defined in Claims 1 to 12 which comprises treating an amine of the formula

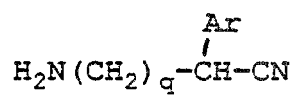

(A)

wherein Ar and q have the meanings defined in Claim 1 as follows:

(a) to form a urea or thiourea containing compound treating an amine of formula (A) with an arylisocyanate or an aryl thioisocyanate of the formula Ar′NCX wherein Ar′ and X have the meanings defined in Claim 1 or a pyrimidine isocyanate or pyrimidine thioisocyanate of the formula

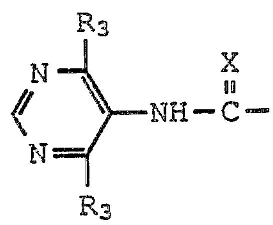

wherein $R_3$ and X have the meanings defined in Claim 1;

(b) to form an amide containing compound acylating an amine of formula (A) with an acid anhydride of the formula $(RCO)_2O$, an acid of the formula $RCO_2H$, or an acid halide of the formula RCOhalo wherein R has the meaning defined in Claim 1 and halo is halogen;

(c) to form an amine containing compound reducing the amide formed in step (b) above or alternatively alkylating an amine of formula (A) with an aldehyde of the formula WCHO wherein W has the meaning defined in Claim 1 to form an imine which is reduced to the amine;

(d) to form compounds of Claim 1 wherein R represents an alkyl group having from 1 to 6 carbon atoms wherein the terminal carbon is substituted with halogen, methoxy or $NR_2R_3$ wherein $R_2$ and $R_3$ have the meanings defined in Claim 1, acylating an amine of formula (A) using an ω-bromoacyl chloride to afford a compound wherein R is $(CH_2)_mBr$ wherein m has the meaning defined in Claim 1 and subjecting said compound to an appropriate nucleophilic substitution to afford the corresponding ω-substituted compounds;

(e) hydrogenating the nitrile moiety of the intermediates obtained in steps (a) through (c) to the corresponding amine and subjecting said amine to steps (a) through (d);

(f) to form an N-oxide treating a compound obtained in (e) above with nitrogen; and

(g) to obtain a pharmaceutically acceptable salt thereof treating the free base obtained in (e) or (f) above with a pharmaceutically acceptable acid.

European Patent Office

# EUROPEAN SEARCH REPORT

Application Number

EP 90121140.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - A2 - 0 335 374 (WARNER-LAMBERT COMPANY) * Claims 1,3-5 * -- | 1,13-15 | C 07 C 275/28<br>C 07 C 309/51<br>C 07 D 213/56<br>C 07 D 307/68<br>C 07 D 239/26<br>A 61 K 31/17 |
| A | EP - A1 - 0 007 184 (AMERICAN HOME PRODUCTS) * Claims 1,8,13 * ---- | 1,13,15 | |

TECHNICAL FIELDS SEARCHED (Int. Cl.5)

C 07 C 275/00
C 07 C 309/00

The present search report has been drawn up for all claims

| Place of search | Date of completion of the search | Examiner |
|---|---|---|
| VIENNA | 11-02-1991 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document